(19) Europäisches Patentamt
European Patent Office
Office européen des brevets

(11) Veröffentlichungsnummer: **0 059 307 A1**

# (12) EUROPÄISCHE PATENTANMELDUNG

(21) Anmeldenummer: 82100317.5

(22) Anmeldetag: 18.01.82

(51) Int. Cl.³: C 07 D 307/935

(30) Priorität: 26.02.81 DE 3107248

(43) Veröffentlichungstag der Anmeldung: 08.09.82 Patentblatt 82/36

(84) Benannte Vertragsstaaten: AT BE CH DE FR GB IT LI LU NL SE

(71) Anmelder: Grünenthal GmbH
Patentabteilung Steinfeldstrasse 2
D-5190 Stolberg(DE)

(72) Erfinder: Vollenberg, Werner, Dr.
Eupener Strasse 26A
D-5190 Stolberg(DE)

(72) Erfinder: Böhlke, Horst, Dr.
Oststrasse 44
D-5190 Stolberg(DE)

## (54) Verfahren zur Herstellung von 2-Oxabicyclo(3.3.0)octanderivaten und danach erhältliche Produkte.

(57) Die Erfindung betrifft ein Verfahren zur Herstellung von wertvolle Zwischenprodukte für pharmakologisch wirksame Substanzen darstellenden 2-Oxabicyclo[3.3.0]octanderivaten der allgemeinen Formel:

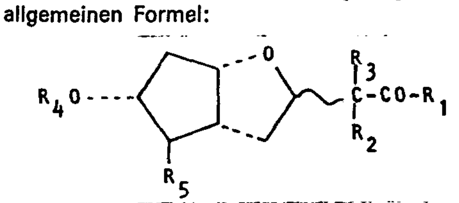

1

worin $R_1$ bis $R_3$ und $R_5$ organische Reste und $R_2$ und $R_3$ darüberhinaus auch Wasserstoff bedeuten und $R_4$ für einen Acylrest oder für eine drei organische Reste tragende Silylgruppe steht, durch Umsetzung einer Verbindung der Formel

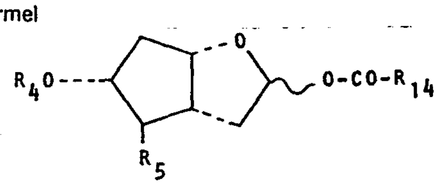

11

worin $R_{14}$ ein niederer Alkylrest oder ein Phenylrest ist mit einer Verbindung der Formel

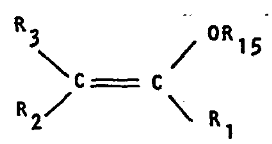

111

worin $R_{15}$ für eine Trimethylsilyl- oder eine niedere Alkanoylgruppe steht, in einem inerten Lösungsmittel in Gegenwart von einer oder mehreren Lewis-Säuren bei Temperaturen zwischen −80°C und +60°C und die danach erhältlichen Produkte.

EP 0 059 307 A1

Croydon Printing Company Ltd.

Patentanmeldung der Grünenthal GmbH, D-5190 Stolberg

Verfahren zur Herstellung von 2-Oxabicyclo[3.3.0]octanderivaten und danach erhältliche Produkte.

Die vorliegende Erfindung bezieht sich auf ein Verfahren zur Herstellung von 2-Oxabicyclo[3.3.0]octanderivaten sowie auf danach erhältliche Produkte. Diese Verbindungen stellen wertvolle Zwischenprodukte zur Herstellung von pharmakologisch wirksamen Substanzen dar. Die erfindungsgemäß erhältlichen 2-Oxabicyclo[3.3.0]octanderivate entsprechen der allgemeinen Formel:

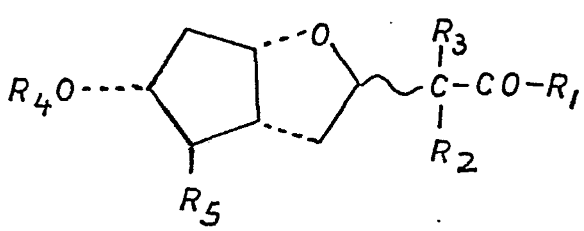

I

Darin bedeutet:

$R_1$ einen Alkylrest mit 1 bis 5 Kohlenstoffatomen, vorzugsweise einen Methyl- oder Ethylrest, oder die Gruppe

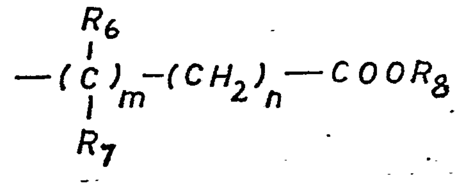

worin $R_6$ und $R_7$ gleich oder verschieden sind und für Wasserstoff oder Alkylreste mit 1 bis 4 Kohlenstoffatomen oder gemeinsam für die Gruppe $-(CH_2)_p-$ stehen und m eine der Zahlen Null oder 1, n eine der Zahlen Null, 1, 2 oder 3 und p eine der Zahlen 4, 5 oder 6 ist und $R_8$ einen leicht abspaltbaren Esterrest bedeutet,

oder einen Rest $-(CH_2)_q-OR_9$, in dem q eine der Zahlen 1, 2 oder 3 ist und $R_9$ eine Acetyl-, Benzoyl-, Trimethylsilyl-, tert.Butyl-dimethyl- oder tert.Butyl-diphenylsilyl-Gruppe ist,

oder einen gegebenenfalls durch eine Methyl-, Ethyl-, Trifluormethyl- oder Methoxygruppe oder durch eine Gruppe $OR_9$, in der $R_9$ die gleiche Bedeutung wie vorstehend hat, durch Fluor, Chlor oder Brom oder durch die Gruppe

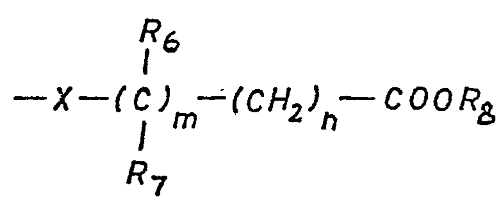

worin X eine einfache Bindung oder ein Sauerstoffatom ist und $R_6$, $R_7$, $R_8$, m und n die gleiche Bedeutung wie vorstehend haben, substituierten Phenylrest

oder eine Methoxy-, Ethoxy- oder Trimethylsilyloxygruppe

oder zusammen mit $R_3$ die Gruppe $-(CH_2)_r-$, in der r eine ganze Zahl von 3 bis 5 ist,

$R_2$ ein Wasserstoffatom, einen Alkylrest mit 1 bis 4 Kohlenstoffatomen oder die Gruppe $-(CH_2)_s-COOR_8$, in der $R_8$ die gleiche Bedeutung wie oben hat und s für Null, 1 oder 2 steht,

$R_3$ ein Wasserstoffatom, einen Alkylrest mit 1 bis 4 Kohlenstoffatomen oder gemeinsam mit $R_1$ die oben (bei $R_1$) definierte Gruppe $-(CH_2)_r-$,

$R_4$ einen Acylrest, vorzugsweise einen niederen Alkanoylrest mit 1 bis 5 Kohlenstoffatomen, insbesondere den Acetylrest, den Benzoylrest oder einen substituierten Benzoylrest, insbesondere den 4-Phenyl-benzoylrest, oder einen Tri-nieder-alkylsilylrest, insbesondere einen Trimethylsilyl- oder tert.Butyl-dimethylsilylrest, oder einen tert.Butyl-diphenylsilyl- oder einen Triphenylsilylrest,

$R_5$ die Gruppe $-CH_2-O-R_4'$ oder die Gruppe

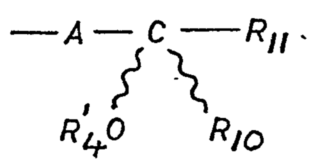

worin $R_4'$ jeweils im Rahmen der Definition von $R_4$ gleich oder verschieden zu $R_4$ ist und worin A für $-CH_2-CH_2-$, (trans)-CH=CH- oder für -C≡C- steht, $R_{10}$ für Wasserstoff, Methyl-, Ethyl- oder Trifluormethyl steht und $R_{11}$ einen Alkylrest

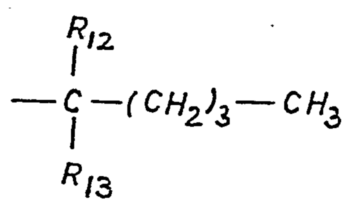

in dem $R_{12}$ und $R_{13}$ gleich oder verschieden sind und für Wasserstoff, Methyl oder Ethyl stehen, oder einen Cyclo-

hexylrest

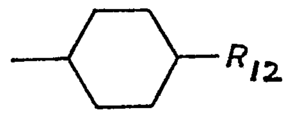

in dem $R_{12}$ die gleiche Bedeutung wie vorstehend hat, bedeutet.

Vorzugsweise stehen in den Verbindungen der Formel I die Reste

$R_2$ und $R_3$ für Wasserstoff oder Methylgruppen und/oder

$R_4$ für die Benzoylgruppe und/oder

$R_6$ und $R_7$ für Wasserstoffatome oder Methylgruppen und/oder

$R_8$ für einen Methyl- oder Ethylrest.

Weiterhin wird bevorzugt, daß die Reste $R_{10}$, $R_{12}$ und $R_{13}$ für Wasserstoffatome stehen, sowie, daß A die Gruppe $-CH_2-CH_2-$ darstellt.

Eine bevorzugte Gruppe von Verbindungen der Formel I entspricht der Formel

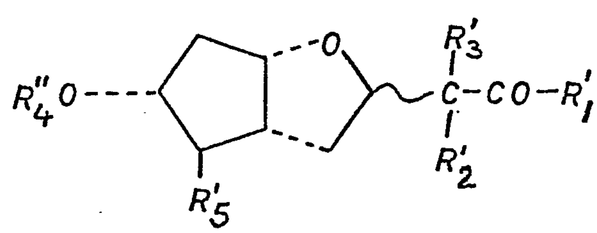

Ia

worin

$R'_1$ einen Methyl- oder Ethylrest oder die Gruppe

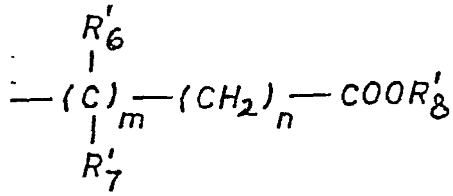

in der $R'_6$ und $R'_7$ gleich oder verschieden sind und für Wasserstoffatome oder Methylgruppen stehen, m eine der Zahlen Null oder 1, n eine der Zahlen Null, 1, 2 oder 3 und $R'_8$ einen Methyl- oder Ethylrest bedeutet

oder einen gegebenenfalls durch eine Methyl-, Ethyl-, Trifluormethyl- oder Methoxygruppe oder durch eine Gruppe $OR_9$, in der $R_9$ die gleiche Bedeutung wie oben hat, durch ein Fluor-, Chlor- oder Bromatom oder durch die Gruppe

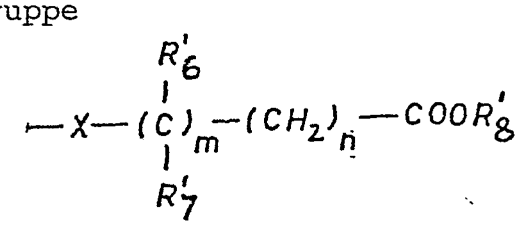

worin X, $R'_6$ bis $R'_8$, m und n die gleiche Bedeutung wie vorstehend haben, substituierten Phenylrest

oder eine Methoxy-, Ethoxy- oder Trimethylsilyloxygruppe

oder zusammen mit $R'_3$ die Gruppe $-(CH_2)_r-$, in der r eine ganze Zahl von 3 bis 5 ist, bedeutet,

$R'_2$ ein Wasserstoffatom, einen Methylrest oder die Gruppe $-(CH_2)_s-COOR'_8$, in der s und $R'_8$ die gleiche Bedeutung wie oben haben, darstellt,

$R'_3$ ein Wasserstoffatom, einen Methylrest oder gemeinsam mit $R'_1$ die oben definierte Gruppe $-(CH_2)_r-$ bedeutet,

$R_4''$ für einen niederen Alkanoylrest mit 1 bis 5 Kohlenstoffatomen, insbesondere den Acetylrest, oder für einen Benzoylrest, einen 4-Phenyl-benzoylrest, einen Trimethylsilyl-, tert.Butyl-dimethylsilyl- oder einen tert.Butyl-diphenylsilylrest steht und

$R_5'$ die Gruppe $-CH_2-O-R_4''$ oder die Gruppe

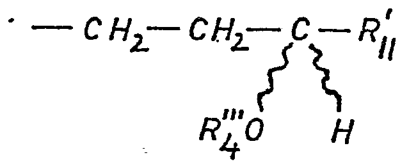

worin $R_4'''$ jeweils im Rahmen der Definition von $R_4'$ gleich oder verschieden zu $R_4''$ ist und $R_{11}'$ für einen n-Pentyl- oder für einen Cyclohexylrest steht, bedeutet.

Vorzugsweise bedeuten $R_1$ bzw. $R_1'$ einen in der definierten Weise substituierten Phenylrest und eine besonders bevorzugte Gruppe von Verbindungen der Formel I entspricht der Formel

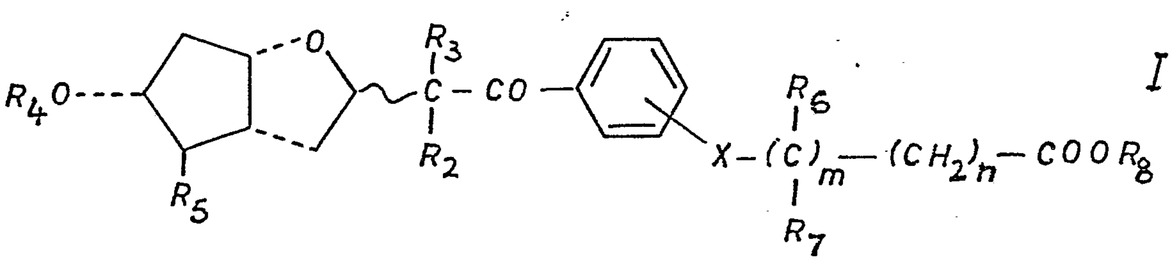

Ib

in der $R_2$ bis $R_8$, X sowie m und n die gleiche Bedeutung wie oben haben, wobei hier insbesondere X eine einfache Bindung darstellt und m und n für Null stehen.

Im letzteren Falle entsprechen die Verbindungen dann insbesondere der Formel

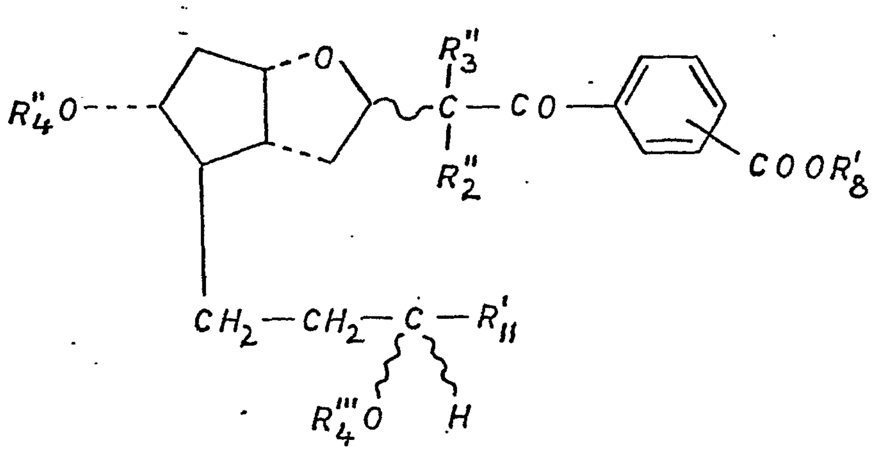

Ic

worin $R''_4$ , $R'''_4$ , $R'_8$ und $R'_{11}$ die gleiche Bedeutung wie oben haben und $R''_2$ und $R''_3$ gleich oder verschieden sind und für Wasserstoffatome oder Methylgruppen stehen.

In den Formelbildern bedeutet eine gepunktete Linie (...) einen Substituenten, in der der endo-Konfiguration (α-Form) an das 2-Oxabicyclo[3.3.0]octan-gerüst der Formel

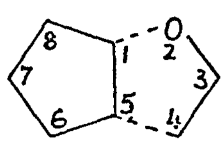

gebunden ist, oder einen Substituenten, der in der S-Konfiguration an eine Kette gebunden ist.

Eine ausgezogene Linie (——) bedeutet, daß ein Substituent in der exo-Konfiguration (ß-Form) vorliegt oder, daß ein solcher in der R-Konfiguration an eine Kette gebunden ist.

Eine Wellenlinie (∼) zeigt an, daß der Substituent stereochemisch nicht definiert ist, d. h. eine solche Gruppe kann an das 2-Oxabicyclo[3.3.0]octan-gerüst in der α- oder ß-Konfiguration oder an einer Kette in der R- oder S-Konfiguration (bzw. jeweils in Gemischen dieser Isomeren) vorliegen.

In den Verbindungen der allgemeinen Formel I liegt die Ringbindung zwischen dem Cyclopentanring und dem Tetrahydrofuranring in der cis-Form vor.

Das erfindungsgemäße Verfahren zur Herstellung der Verbindungen der Formel I ist dadurch gekennzeichnet, daß eine Verbindung der Formel

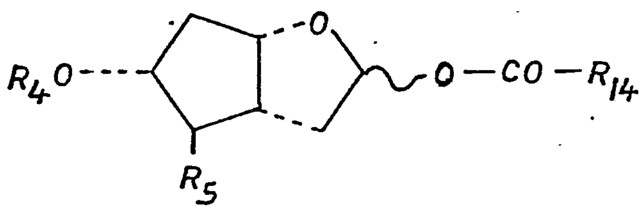

II

worin $R_4$ und $R_5$ die gleiche Bedeutung wie in Formel I haben und $R_{14}$ für einen Alkylrest mit 1 bis 4 Kohlenstoffatomen, insbesondere die Methylgruppe, oder für einen Phenylrest steht,

mit einer Verbindung der Formel

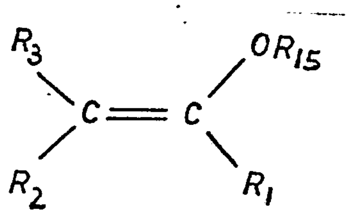

III

worin $R_1$, $R_2$ und $R_3$ die gleiche Bedeutung wie in Formel I haben und $R_{15}$ für eine Trimethylsilyl- oder eine niedere Alkanoylgruppe, vorzugsweise die Acetylgruppe, steht,

in einem inerten Lösungsmittel in Gegenwart von einer oder mehreren Lewis-Säuren bei Temperaturen zwischen -80°C und +60°C umgesetzt wird.

Als Lewis-Säuren kommen dabei insbesondere solche aus der Gruppe Aluminiumchlorid, Bortrifluorid, Kupfer-II-sulfat,

Silberperchlorat, Silbertetrafluoroborat, Titan-IV-chlorid, Zinkbromid, Zinkchlorid oder Zinn-IV-chlorid in Betracht. Vorzugsweise werden Bortrifluorid (z. B. in Form seines Etherates) sowie Zinn-IV-chlorid verwendet. Generell kommen Mengen von $10^{-3}$ Mol bis $10^{2}$ Mol, im allgemeinen äquivalente Mengen Lewissäure pro Mol eingesetzter Verbindung der allgemeinen Formel II zur Anwendung.

Die Reaktionstemperaturen liegen vorzugsweise zwischen -5°C und +20°C.

Bei den einzusetzenden inerten Lösungsmitteln handelt es sich insbesondere um lineare oder cyclische Ether (z. B. Diethylether, Dimethoxyethan, Dioxan oder Tetrahydrofuran), bei der Reaktionstemperatur flüssige aliphatische oder aromatische Kohlenwasserstoffe (z. B. n-Hexan, Benzol oder Toluol) oder niedere chlorierte aliphatische Kohlenwasserstoffe (z. B. Methylenchlorid, Chloroform oder Tetrachlorkohlenstoff) usw.

Die bei der Synthese anfallenden Diastereomerengemische der Verbindungen der Formel I lassen sich auf bekannte Weise, vornehmlich durch Säulenchromatographie, isolieren. Durch Umkristallisation oder Säulenchromatographie (insbesondere an Kieselgel) können die Diastereomerengemische auf üblichem Wege getrennt werden. Die Erfindung bezieht sich sowohl auf die reinen Diastereomeren als auch auf deren Gemische bzw. deren Herstellung.

Durch das erfindungsgemäße Verfahren wird ein neuer, hinsichtlich seiner Durchführbarkeit nicht vorhersehbarer Weg zur Herstellung der als Zwischenprodukte zur Gewinnung pharmakologisch wirksamer Substanzen wertvollen Verbindungen der Formel I geschaffen. Diese sind überwiegend neu und ließen sich, soweit sie bereits bekannt sind, bisher nur schwierig und/oder mit schlechten Ausbeuten erhalten. Demgegenüber erlaubt es das erfindungsgemäße Verfahren, diese Verbindungen rasch in hohen Ausbeuten auf einfache

Weise herzustellen. Speziell durch die leichte Zugänglichkeit und die große Variationsbreite der Verbindungen der Formel III ist das erfindungsgemäße Verfahren den bekannten bzw. bisher benutzten Verfahrensweisen technisch in hohem Maße überlegen. Durch seine breite Anwendbarkeit eröffnet das erfindungsgemäße Verfahren auch den Zugang zu zahlreichen, bisher nicht herstellbaren Verbindungen der Formel I bzw. zu den daraus erhältlichen pharmakologisch wirksamen Verbindungen.

Die Verbindungen der Formel I lassen sich in an sich bekannter Weise durch Abspalten des Restes $R_4$ (d. h. dessen Ersatz durch ein Wasserstoffatom) und gegebenenfalls Ersatz eines in ihnen vorhandenen Restes $R_8$ durch ein pharmazeutisch verträgliches Kation oder durch ein Wasserstoffatom bzw. Ersatz von $R_9$ und/oder $R'_4$ durch Wasserstoff insbesondere dann in pharmakologisch wirksame Substanzen überführen, wenn $R_5$ von $-CH_2-O-R'_4$ verschieden ist.

Steht dagegen $R_5$ in den Verbindungen der Formel I für die Gruppe $-CH_2-OR'_4$, so wird zur Gewinnung der pharmakologisch wirksamen Verbindungen in an sich bekannter Weise diese Gruppe zunächst in den Rest $-CH_2-OH$ überführt und (wenn $R_1$ nicht für eine Methoxy-, Ethoxy- oder Trimethylsilyloxygruppe steht) die den Rest $R_1$ tragende Carbonylgruppe z. B. durch Ketalisierung mit Ethylenglykol oder Propylenglykol intermediär geschützt. In dem so erhaltenen Derivat wird dann die in 6-Stellung des 2-Oxabicyclo[3.3.0]octan-gerüstes stehende Hydroxymethylgruppe zur Aldehydgruppe oxydiert und diese dann beispielsweise analog zu dem in der DE-OS 2 757 919 beschriebenen Vorgehen in die Gruppe $-A-(COH-R_{10})-R_{11}$ worin A, $R_{10}$ und $R_{11}$ die gleiche Bedeutung wie oben haben, überführt. Durch Austausch der Reste $R_4$, $R_8$ und $R_9$ gegen Wasserstoff (bzw. $-R_8-$ gegen ein Kation) und Freisetzung der den Rest $R_1$ tragenden Carbonylgruppe erhält man dann Verbindungen der Formel

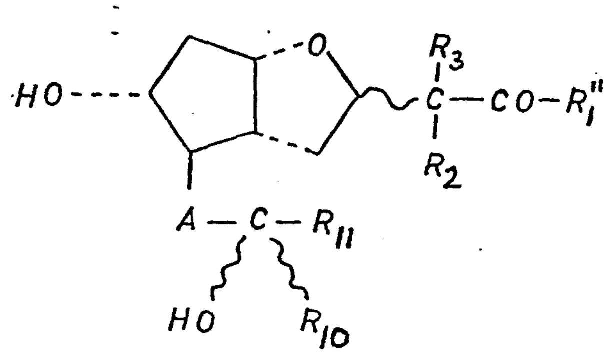

(a)

in der $R_2$, $R_3$, $R_{10}$, $R_{11}$ und A die gleiche Bedeutung wie oben haben und $R_1''$ dem Rest $R_1$ entspricht, mit der Maßgabe, daß darin enthaltene Reste $R_8$ und $R_9$ auch für Wasserstoff bzw. ($R_8$) für ein Kation stehen können und daß $R_1''$ auch eine Hydroxylgruppe sein kann.

In den Verbindungen der Formel (a) kann dann z. B. in der aus der DE-OS 28 53 849 bekannten Weise die den Rest $R_1''$ tragende Carbonylgruppe zu einer CHOH- oder $CH_2$-Gruppe reduziert werden, wobei man zu pharmakologisch wirksamen Verbindungen der Formel

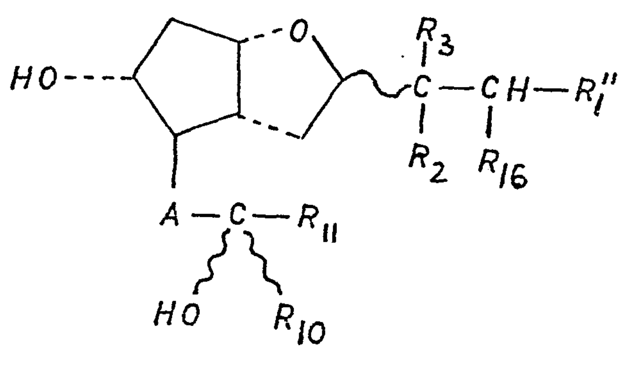

(b)

worin $R_1''$ , $R_2$, $R_3$, $R_{10}$, $R_{11}$ und A die gleiche Bedeutung wie oben haben und $R_{16}$ für eine Hydroxylgruppe oder für ein Wasserstoffatom steht,

gelangt.

Die Ausgangsverbindungen für das erfindungsgemäße Verfahren sind zum Teil bereits bekannt, im übrigen können sie analog zu Literaturvorschriften wie folgt erhalten werden.

Zur Herstellung der Verbindungen der allgemeinen Formel II setzt man Lactole der Formel

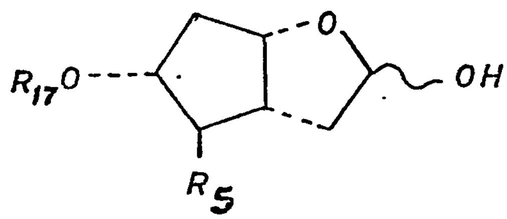

(c)

worin $R_5$ die gleiche Bedeutung wie oben hat und $R_{17}$ die gleiche Bedeutung wie $R_4$ hat, außerdem aber auch für Wasserstoff stehen kann

mit Acylchloriden/Pyridin in Gegenwart von z. B. Essigsäureethylester oder z. B. auch mit Säureanhydriden/Pyridin in Tetrahydrofuran um und bringt die so gebildeten Verbindungen der allgemeinen Formel II dann ohne weitere Reinigung mit Verbindungen der allgemeinen Formel III zur Reaktion.

Die Lactole der Formel (c) werden gemäß E. W. Yankee, U. Axen und G. L. Bundy (J.Am.Chem.Soc. 96, 5865 (1974)) oder auf analoge Weise durch Reduktion der entsprechenden Lactone der allgemeinen Formel

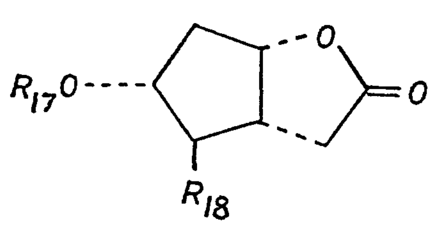

(d)

worin $R_{17}$ die gleiche Bedeutung wie in Formel (c) hat und $R_{18}$ eine Gruppe der Formel $-CH_2-O-R'_{17}$ , der Formel

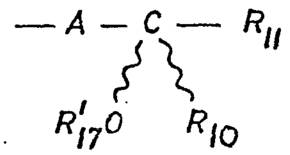

oder der Formel $-A-CO-R_{11}$ ist, worin A, $R_{10}$ und $R_{11}$ die gleiche Bedeutung wie oben haben und $R'_{17}$ im Rahmen der Definitionen von $R_{17}$ gleich oder verschieden zu $R_{17}$ ist, z. B. mittels Diisobutylaluminiumhydrid bei ca. -78°C in einem Gemisch von Toluol/Tetrahydrofuran hergestellt.

Die Ausgangslactone der Formel (d) lassen sich entsprechend Literaturangaben bzw. in dazu analoger Weise erhalten. Es wird hierzu beispielsweise verwiesen auf:

Bindra und Bindra, "Prostaglandin Synthesis", Academic Press Inc., New York, San Francisco, London 1977

oder

A. Mitra, "The Synthesis of Prostaglandins", John Wiley and Sons, New York, London, Sydney, Toronto 1977

Die Verbindungen der allgemeinen Formel III können ebenfalls entsprechend Literaturangaben bzw. in analoger Weise hergestellt werden. Insbesondere sei auf folgende Literaturstellen hingewiesen:

1) Methoden der organischen Chemie (Houben Weyl) Bd. VI/1d, Seite 104, Georg Thieme Verlag, Stuttgart 1978

2) Methoden der organischen Chemie (Houben-Weyl) Bd. VII/2b, Seite 1906, Georg Thieme Verlag, Stuttgart 1976

3) J. K. Rasmussen, Synthesis 91 (1977)

4) R. D. Miller und D. R. McKean, Synthesis 730 (1979)

5) J. Fleming und J. Paterson, Synthesis 736 (1979)

6) A. Wissner, J.Org.Chem. 44, 4619 (1979)

7) H. O. House, L. J. Czuba, M. Gall und H. D. Olmstead, J.Org.Chem. 34, 2324 (1969).

Die folgenden Beispiele dienen zur näheren Erläuterung der Erfindung. Alle Temperaturangaben darin sind unkorrigiert.

Die $^{1}H$-NMR-Spektren wurden bei 60 MHz und die $^{13}C$-NMR-Spektren bei 15,08 MHz mit dem Gerät WP-60 der Firma Bruker aufgenommen. Die chemische Verschiebung der spektroskopischen Resonanzdaten wurde in ppm gemessen.

Die Reaktionen wurden dünnschichtchromatographisch verfolgt (Fertigplatten, Kieselgel 60, E. Merck). Für die Säulenchromatographie wurde Kieselgel 60 (0,063 - 0,200 mm oder auch 0,040 - 0,063 mm) E. Merck, benutzt.

## Beispiel 1

3α,ß-(1RS-Cyclopentan-2-on-1-yl)-6ß-(3RS-acetoxy-n-octyl) 7α-benzoyloxy-2-oxabicyclo[3.3.0]octan

700 mg 3α,ß-Acetoxy-6ß-(3RS-acetoxy-n-octyl)-7α-benzoyloxy-2-oxabicyclo[3.3.0]octan und 278 mg (1-Cyclopenten-1-yl-oxy)-trimethylsilan (H. O. House et al., J.Org.Chem. 34, 2324 (1969)) werden in 10 ml absolutem Methylenchlorid gelöst. Zu der Lösung gibt man unter Rühren bei einer Temperatur von 0° bis +5°C innerhalb von 15 Minuten tropfenweise eine Lösung von 0,18 ml Zinn-IV-chlorid in 10 ml Methylenchlorid. Der Reaktionsverlauf wird dünnschichtchromatographisch verfolgt (Toluol/Diethylether 1 : 8).

Nach Zugabe der Lewissäure wird noch 1 Stunde bei der gleichen Temperatur nachgerührt. Dann wird die kalte Lösung unter Rühren tropfenweise so in gesättigte Natriumhydrogencarbonatlösung gegeben, daß der pH-Wert bei etwa 7 gehalten wird.

Die organische Phase wird mit 10 ml Methylenchlorid verdünnt, gut durchgeschüttelt und von der wässrigen Phase abgetrennt. Die wässrige Phase wird noch zweimal mit 5 ml Methylenchlorid extrahiert und die vereinigten Extrakte werden zweimal mit je 3 ml gesättigter Natriumchloridlösung ausgewaschen. Das Lösungsmittel wird nach dem Trocknen über Natriumsulfat im Vakuum abdestilliert.

Man erhält 698 mg der Titelverbindung in Form eines farblosen Öls.

| | |
|---|---|
| $^{1}$H-NMR($CDCl_3$): | 0,60 - 1,03 (m,3H) |
| | 1,99; 2,00 (2s,3H) |
| | 4,13 - 5,10 (m,4H) |
| | 7,23 - 8,13 (m,5H) |
| $^{13}$C-NMR (Methanol-$d_4$): | 51,25; 52,95; 74,86; |
| | 75,11; 81,19; 83,38; |
| | 220,45. |

<u>Beispiel 2</u>

Man verfährt wie in Beispiel 1, jedoch unter Verwendung einer Lösung von 0,455 ml Bortrifluorid-Etherat (50 % $BF_3$ in Diethylether) in 10 ml Methylenchlorid anstelle der Zinn-IV-chloridlösung und erhält 682 mg des Produktes in Form eines farblosen Öls, dessen spektroskopische Resonanzdaten mit denen des nach Beispiel 1 hergestellten Produktes identisch sind.

Das in den Beispielen 1 und 2 als Ausgangsprodukt verwendete Halbacetal-Acylal erhält man wie folgt:

a) 3α,ß-Hydroxy-6ß-(3RS-hydroxy-n-octyl)-7α-benzoyloxy-2-oxabicyclo[3.3.0]octan

Eine Lösung von 5 g 3-Oxo-6ß-(3-oxo-n-octyl)-7α-benzoyloxy-2-oxabicyclo[3.3.0]octan (W. Skuballa et al., J.Med.Chem. 21, 443 (1978)) in 100 ml absolutem Tetrahydrofuran wird bei -78°C unter gutem Rühren und Überleiten von trockenem Stickstoff tropfenweise vorsichtig mit 100 ml einer Lösung von Diisobutylaluminiumhydrid (ca. 0,6 Mol/l) in Toluol versetzt. Nach erfolgter Zugabe wird noch etwa 30 Minuten bei -78°C weitergerührt.

Der Reaktionsverlauf wird dünnschichtchromatographisch verfolgt (Toluol/Aceton - 1 : 1).

Das Reaktionsgemisch wird nach Beendigung der Reaktion unter weiterer Kühlung vorsichtig tropfenweise mit 100 ml gesättigter Ammoniumchloridlösung versetzt. Man erwärmt anschließend langsam auf Raumtemperatur, filtriert über ca. 30 g vorher mit Essigsäureethylester ausgewaschene Filtererde und spült noch viermal mit je 50 ml Essigsäureethylester nach. Das Filtrat bildet zwei Schichten, die getrennt werden. Die organische Schicht wird zweimal mit je 30 ml gesättigter Natriumchloridlösung gewaschen und über Natriumsulfat getrocknet. Nach Abdampfen der Lösungsmittel am Rotationsverdampfer hinterbleiben 4,6 g eines fast farblosen Öls.

Durch Säulenchromatographie mit Toluol/Aceton (1 : 1) erhält man 4,5 g der Titelverbindung in Form eines farblosen Öls.

$^1$H-NMR($CDCl_3$):

0,70 - 1,17 (m,3H)
3,30 - 3,80 (m,1H)
4,56 - 5,26 (m,2H)
5,40 - 5,80 (m,1H)
7,07 - 8,17 (m,5H)

b) 3α,ß-Acetoxy-6ß-(3RS-acetoxy-n-octyl)-7α-benzoyloxy-2-oxa-bicyclo[3.3.0]octan

1,32 g des in Stufe a) erhaltenen Produktes werden in 3,3 ml absolutem Tetrahydrofuran und 3,3 ml absolutem Pyridin gelöst. In die Lösung gibt man unter Eiswasserkühlung langsam tropfenweise 3,3 ml Essigsäureanhydrid. Man läßt zunächst noch 1 Stunde im Eisbad und anschliessend etwa 15 Stunden bei Raumtemperatur stehen.

Der Reaktionsverlauf wird dünnschichtchromatographisch verfolgt (Methylenchlorid/Aceton - 4 : 1).

Die leichter flüchtigen Bestandteile werden unter Feuchtigkeitsausschluß bei 10 - 12 Torr abdestilliert. Der viskose Rückstand wird so lange im Hochvakuum am Rotationsverdampfer bei 40° bis 45°C Badtemperatur erhitzt, bis alle flüchtigen Bestandteile entfernt sind.

Man erhält 1,56 g der Titelverbindung in Form eines farblosen Öls.

$^1$H-NMR($CDCl_3$):

0,60 - 1,06 (m,3H)
2,03; 2,06 (2s,6H)
4,57 - 5,20 (m,3H)
6,17 - 6,47 (m,1H)
7,20 - 8,03 (m,5H)

Beispiel 3

3α,ß-(1RS-Cyclopentan-2-on-1-yl)-6ß-acetoxymethyl-7α-benzoyloxy-2-oxabicyclo[3.3.0]octan

10,0 g 3α,ß-Acetoxy-6ß-acetoxymethyl-7α-benzoyloxy-2-oxabicyclo[3.3.0]octan und 4,3 g (1-Cyclopenten-1-yloxy)-trimethylsilan werden in 200 ml absolutem Methylenchlorid gelöst und unter Rühren bei einer Temperatur von 0° bis +5°C tropfenweise mit einer Lösung von 0,83 ml Bortrifluorid-Etherat (50 % $BF_3$ in Diethylether) in 20 ml Methylenchlorid versetzt. Der Reaktionsverlauf wird dünnschichtchromatographisch verfolgt (Toluol/Diethylether 1 : 8). Nach der Zugabe wird 30 Minuten in der Kälte weitergerührt und dann die noch kalte Lösung unter Rühren tropfenweise so in gesättigte Natriumhydrogencarbonatlösung gegeben, daß der pH-Wert bei etwa 7 bleibt. Die organische Phase wird mit 500 ml Methylenchlorid verdünnt, gut durchgeschüttelt und von der wässrigen Phase abgetrennt. Die wässrige Phase wird noch zweimal mit je 50 ml Methylenchlorid extrahiert und dann werden die vereinigten Extrakte zweimal mit je 20 ml gesättigter Natriumchloridlösung gewaschen. Es wird über Natriumsulfat getrocknet. Nach Abdampfen des Lösungsmittels erhält man 10,5 g eines hellgelben Öls.

Durch Säulenchromatographie mit Toluol/Diethylether (1 : 8) erhält man 9,4 g der Titelverbindung in Form eines farblosen Öls.

| | |
|---|---|
| $^1$H-NMR($CDCl_3$): | 2,00 (m,3H) |
| | 3,97 - 4,20 (d,2H) |
| | 4,34 - 5,30 (m,3H) |
| | 7,33 - 8,20 (m,5H) |

$^{13}$C-NMR($CDCl_3$): 49,27; 52,05; 76,58; 77,71; 81,83; 81,96; 220,00.

Beispiel 4

Man verfährt wie in Beispiel 3, jedoch unter Verwendung einer Lösung von 3,3 ml Zinn-IV-chlorid in 20 ml Methylenchlorid anstelle der Bortrifluorid-Etherat-Lösung und erhält 9,0 g der gleichen Verbindung wie in Beispiel 3, deren spektroskopische Resonanzdaten mit den in Beispiel 3 genannten identisch sind.

Das in den Beispielen 3 und 4 als Ausgangsprodukt verwendete Halbacetal-Acylal kann man wie folgt in zweistufiger Synthese (c und d) herstellen:

c) 3α,ß-Hydroxy-6ß-hydroxymethyl-7α-benzoyloxy-2-oxabicyclo[3.3.0]octan

Eine Lösung von 5 g 3-Oxo-6ß-hydroxymethyl-7α-benzoyloxy-2-oxabicyclo[3.3.0]octan in 200 ml absolutem Tetrahydrofuran wird bei -78°C unter gutem Rühren und Überleiten von trockenem Stickstoff tropfenweise mit 75 ml einer Lösung von Diisobutylaluminiumhydrid (ca. 1,2 Mol/l) in Toluol, vorsichtig versetzt. Man verfährt weiter in der in Beispiel 2 bei a) angegebenen Weise und erhält so 4,9 g der Titelverbindung als nahezu farbloses Öl, das ohne weitere Reinigung umgesetzt werden kann.

Durch Säulenchromatographie mit Methylenchlorid/Aceton (4 : 1) erhält man die Titelverbindung in Form eines farblosen Öls.

| | |
|---|---|
| $^1$H-NMR($CDCl_3$): | 3,16 - 3,28 (d,2H) |
| | 4,47 - 5,80 (m,3H) |
| | 7,23 - 8,17 (m,5H) |

d) 3α,ß-Acetoxy-6ß-acetoxymethyl-7α-benzoyloxy-2-oxabicyclo[3.3.0]octan

Eine Lösung von 1 g des in Stufe c erhaltenen Produktes

in 3 ml absolutem Tetrahydrofuran wird mit 4 ml absolutem Pyridin versetzt, und nach Abkühlung im Eisbad werden 4 ml Essigsäureanhydrid tropfenweise hinzugefügt. Man läßt 1 Stunde im Eiswasserbad und anschließend noch etwa 15 Stunden bei Raumtemperatur stehen und arbeitet dann in der in Beispiel 2 bei b) beschriebenen Weise auf. Man erhält 1,2 g der Titelverbindung in Form eines farblosen Öls.

| | |
|---|---|
| $^1H$-NMR($CDCl_3$): | 2,00; 2,05 (2s,6H) |
| | 3,97 - 4,20 (d,2H) |
| | 4,53 - 5,40 (m,2H) |
| | 6,17 - 6,47 (m,1H) |
| | 7,20 - 8,10 (m,5H) |
| $^{13}C$-NMR($CDCl_3$): | 64,45; 84,10; 100,35; |
| | 166,10; 170,30; 170,86. |

Beispiel 5

3α,ß-(Propan-2-on-1-yl)-6ß-(3RS-acetoxy-n-octyl)-7α-benzoyloxy-2-oxabicyclo[3.3.0]octan

100 mg 3α,ß-Acetoxy-6ß-(3RS-acetoxy-n-octyl)-7α-benzoyloxy-2-oxabicyclo[3.3.0]octan und 25 mg Essigsäure-α-methylvinyl-ester werden in 3 ml absolutem Methylenchlorid gelöst. Die Lösung wird unter Rühren und Eiswasserkühlung langsam tropfenweise mit einer Lösung von 0,0255 ml Zinn-IV-chlorid in 2 ml Methylenchlorid versetzt. Es wird noch etwa 1 Stunde in der Kälte nachgerührt. Der Reaktionsverlauf wird dünnschichtchromatographisch verfolgt (Diisopropylether/Diethylether 3 : 2).

Die kalte Lösung wird unter gutem Rühren tropfenweise in 20 ml gesättigte Natriumhydrogencarbonatlösung eingetragen und dann wird das Gemisch in der in Beispiel 1 beschriebenen Weise weiter aufgearbeitet wobei man 90 mg eines gelblich gefärbten Öls erhält. Durch dessen Säulenchromatographie mit Diisopropylether/Diethylether (3 : 2) erhält man 60 mg der Titelverbindung in Form eines farblosen Öls.

$^1$H-NMR($CDCl_3$): 0,63 - 1,10 (m,3H)
1,97; 2,00 (2s,3H)
2,16 (s,3H)
4,23 - 5,07 (m,4H)
7,20 - 8,10 (m,5H)

Beispiel 6

Man verfährt wie in Beispiel 5, jedoch unter Verwendung einer Lösung von 0,0065 ml Bortrifluorid-Etherat (50 % $BF_3$ in Diethylether) in 2 ml Methylenchlorid anstelle der Zinn-IV-chloridlösung und erhält 65 mg eines farblosen Öls.

Die spektroskopischen Resonanzdaten sind mit denen des nach Beispiel 5 hergestellten Produktes identisch.

Beispiel 7

3α,ß-(Propan-2-on-1-yl)-6ß-acetoxymethyl-7α-benzoyloxy-2-oxabicyclo[3.3.0]octan

100 mg 3α,ß-Acetoxy-6ß-acetoxymethyl-7α-benzoyloxy-2-oxabicyclo[3.3.0]octan und 30 mg Essigsäure-α-methyl-vinylester, gelöst in 3 ml absolutem Methylenchlorid, werden unter Zusatz einer Lösung von 0,0083 ml Bortrifluorid-Etherat (50 % $BF_3$ in Diethylether) in 2 ml Methylenchlorid entsprechend der in Beispiel 5 beschriebenen Weise umgesetzt und dann aufgearbeitet, wobei man 95 mg der Titelverbindung in Form eines farblosen Öls erhält.

$^1$H-NMR($CDCl_3$): 1,97; 2,00 (2s,3H)
2,16 (s,3H)
3,83 - 5,40 (m,5H)
7,20 - 8,20 (m,5H)

Beispiel 8

3α,ß-[2-(2'-Methoxycarbonylphenyl)-ethan-2-on-1-yl]-6ß-(3RS-acetoxy-n-octyl)-7α-benzoyloxy-2-oxabicyclo[3.3.0]-octan

1 g 3α,ß-Acetoxy-6ß-(3RS-acetoxy-n-octyl)-7α-benzoyloxy-2-oxabicyclo[3.3.0]octan und 0,6 g 1-Trimethylsilyloxy-1-(2'-methoxycarbonyl-phenyl)-ethylen werden in 20 ml absolutem Methylenchlorid gelöst. In die Lösung wird unter Rühren bei 0° bis +5°C vorsichtig tropfenweise eine Lösung von 0,246 ml Zinn-IV-chlorid in 10 ml Methylenchlorid gegeben. Der Reaktionsverlauf wird dünnschichtchromatographisch verfolgt (Diisopropylether/Diethylether 3 : 2). Nach Zugabe der Lewissäure wird noch 1 Stunde in der Kälte nachgerührt und die kalte Reaktionslösung unter Rühren in gesättigte Natriumhydrogencarbonatlösung so eingetragen, daß der pH-Wert bei etwa 7 gehalten wird.

Die organische Phase wird mit 20 ml Methylenchlorid verdünnt, gut durchgeschüttelt und von der wässrigen Phase abgetrennt. Die wässrige Phase wird noch zweimal mit 10 ml Methylenchlorid extrahiert, die vereinigten Extrakte werden zweimal mit 5 ml gesättigter Natriumchloridlösung gewaschen und nach dem Trocknen über Natriumsulfat vom Lösungsmittel befreit. Nach Säulenchromatographie mit Diisopropylether/Diethylether (3 : 2) werden 1,1 g der Titelverbindung in Form eines farblosen Öls erhalten.

| | |
|---|---|
| $^1H$-NMR($CDCl_3$): | 0,57 - 1,06 (m,3H) |
| | 2,00 (s,3H) |
| | 2,93 - 3,27 (m,2H) |
| | 3,77; 3,88 (2s,3H) |
| | 4,33 - 5,08 (m,4H) |
| | 7,23 - 8,09 (m,5H) |
| $^{13}C$-NMR (Aceton-$d_6$): | 48,51; 74,33; 82,16; 168,32; 171,38; 206,80. |

Die im vorstehenden Beispiel als Ausgangsprodukt verwendete Silylverbindung erhält man auf folgende Weise:
In eine Lösung von 17,8 g 2-Acetyl-benzoesäuremethylester (M. S. Newman et al., J.Org.Chem. 27, 863 (1962)) , 16,5 ml

Triäthylamin (über festem Kaliumhydroxid destilliert) und 80 ml Toluol (über Natriumhydrid destilliert) gibt man bei 20 - 25°C tropfenweise unter Rühren 18,4 ml Trifluormethansulfonsäure-trimethylsilylester, gelöst in 10 ml Toluol. Es wird noch 3 Stunden bei dieser Temperatur nachgerührt und die Toluolphase im Scheidetrichter abgetrennt. Das Toluol wird unter Feuchtigkeitsausschluß im Vacuum abgedampft und das zurückbleibende Öl im Vakuum destilliert.

Man erhält 10,2 g 1-Trimethylsilyloxy-1-(2'-methoxycarbonylphenyl)-ethylen vom Siedepunkt 125° - 127°C (11 Torr).

| | |
|---|---|
| $^1$H-NMR($CDCl_3$): | 3,80 (s,3H) |
| | 4,30 - 4,57 (2d,2H) |
| | 7,10 - 7,80 (m,4H) |

Beispiel 9

3α,ß-[2-(2'-Methoxycarbonylphenyl)ethan-2-on-1yl]-6ß-acetoxymethyl-7α-benzoyloxy-2-oxabicyclo[3.3.0]octan

100 mg 3α,ß-Acetoxy-6ß-acetoxymethyl-7α-benzoyloxy-2-oxabicyclo[3.3.0]octan und 70 mg 1-Trimethylsilyloxy-1-(2'-methoxycarbonylphenyl)-ethylen werden in 3 ml absolutem Methylenchlorid gelöst. In die Lösung gibt man unter Rühren bei 0° bis 5°C tropfenweise eine Lösung von 0,0033 ml Zinn-IV-chlorid in 3 ml Methylenchlorid. Der Reaktionsverlauf wird dünnschichtchromatographisch verfolgt (Diisopropylether/Diethylether 3 : 1).

Nach Zugabe der Lewissäure wird noch etwa 15 Minuten in der Kälte weitergerührt und dann entsprechend der in Beispiel 5 beschriebenen Weise aufgearbeitet.

Nach Säulenchromatographie des Rohproduktes mit Diisopropylether/Diethylether (3 : 2) werden 50 mg der Titelverbindung in Form eines farblosen Öls erhalten.

$^1$H-NMR($CDCl_3$):

2,00 (s,3H)
2,91 - 3,23 (m,2H)
3,61 - 5,40 (m,8H)
7,03 - 8,17 (m,4H)

Beispiel 10

Man verfährt wie in Beispiel 9, jedoch unter Verwendung einer Lösung von 0,0083 ml Bortrifluorid-Etherat (50 % $BF_3$ in Diethylether) in 3 ml Methylenchlorid anstelle der Zinn-IV-chloridlösung und erhält nach säulenchromatographischer Reinigung 45 mg des gleichen Produktes wie in Beispiel 9. Die spektroskopischen Resonanzdaten sind mit den in Beispiel 9 genannten identisch.

Beispiel 11

3α,ß-(1RS-Propan-2-on-1-ethoxycarbonyl-1-yl)-6ß-(3RS-acetoxy-n-octyl)-7α-benzoyloxy-2-oxabicyclo[3.3.0]octan

100 mg 3α,ß-Acetoxy-6ß-(3RS-acetoxy-n-octyl)-7α-benzoyloxy-2-oxabicyclo[3.3.0]octan und 44 mg 3-Trimethylsilyloxycrotonsäureethylester (W. Kantlehner et al., Chem.Ber. 105, 2264 (1972)) werden in 3 ml absolutem Methylenchlorid gelöst. Die Lösung wird in einem Kältebad auf -10°C abgekühlt und bei dieser Temperatur vorsichtig unter Rühren mit einer Lösung von 0,0246 ml Zinn-IV-chlorid in 3 ml Methylenchlorid tropfenweise versetzt. Der Reaktionsverlauf wird dünnschichtchromatographisch verfolgt (Diethylether/n-Hexan 2 : 1). Das Reaktionsgemisch wird noch etwa 30 Minuten im Kältebad gehalten und dann entsprechend der in Beispiel 5 beschriebenen Weise aufgearbeitet. Man erhält 110 mg der Titelverbindung in Form eines leicht gelblich gefärbten Öls.

$^1$H-NMR($CDCl_3$): 0,60 - 1,00 (m,3H)
2,00; 2,03 (2s,3H)
2,13 - 2,30 (m,3H)
3,33 - 3,70 (m,1H)
3,90 - 5,27 (m,6H)
7,23 - 8,13 (m,5H)

$^{13}$C-NMR($CDCl_3$): 61,41; 61,53; 64,57; 79,24; 79,85; 201,64.

Beispiel 12

3α,ß-(1RS-Propan-2-on-1-ethoxycarbonyl-1-yl)-6ß-acetoxymethyl-7α-benzoyloxy-2-oxabicyclo[3.3.0]octan

200 mg 3α,ß-Acetoxy-6ß-acetoxymethyl-7α-benzoyloxy-2-oxabicyclo[3.3.0]octan und 112 mg 3-Trimethylsilyloxycrotonsäureethylester werden in 5 ml absolutem Methylenchlorid gelöst. Die Lösung wird im Eiswasserbad abgekühlt und unter Rühren tropfenweise mit einer Lösung von 0,066 ml Zinn-IV-chlorid in 4 ml Methylenchlorid versetzt. Der Reaktionsverlauf wird dünnschichtchromatographisch verfolgt (Diisopropylether/Diethylether 3 : 1). Es wird noch etwa 15 Minuten nachgerührt und dann entsprechend der in Beispiel 1 beschriebenen Weise aufgearbeitet. Nach Säulenchromatogaphie des Rohproduktes mit Diisopropylether-Diethylether (3 : 2) werden 122 mg der Titelverbindung in Form eines farblosen Öls erhalten.

$^1$H-NMR($CDCl_3$): 1,03 - 1,26 (m,3H)
2,00 (s,3H)
2,13 - 2,30 (m,3H)
3,33 - 3,70 (m,1H)
3,90 - 5,27 (m,7H)
7,17 - 8,03 (m,5H)

Beispiel 13

3α,ß-(1RS-3-Methoxycarbonyl-1-benzoyl-propyl-1)-6ß-(3RS-acetoxy-n-octyl)-7α-benzoyloxy-2-oxabicyclo[3.3.0]octan

Zur Lösung von 461 mg 3α,ß-Acetoxy-6ß-(3RS-acetoxy-n-octyl)-7α-benzoyloxy-2-oxabicyclo[3.3.0]octan und 306 mg 5-Trimethylsilyloxy-5-phenyl-4-pentensäuremethylester (R. D. Miller et al., Synthesis 1979, 730) in 5 ml absolutem Methylenchlorid wird unter Eiskühlung und Rühren die Lösung von 0,12 ml Zinn-IV-chlorid in 5 ml absolutem Methylenchlorid tropfenweise zugegeben. Der Reaktionsverlauf wird dünnschichtchromatographisch verfolgt (Diethylether/Diisopropylether/Hexan 1 : 1 : 1). Nach 60 Minuten wird das Reaktionsgemisch unter Eiskühlung tropfenweise so in 30 ml gesättigte Natriumhydrogencarbonatlösung gegeben, daß der pH-Wert stets um 7 bleibt. Die organische Phase wird abgetrennt, die wäßrige Schicht dreimal mit wenig Methylenchlorid nachextrahiert und schließlich werden die vereinigten organischen Phasen einmal mit 20 ml gesättigter Natriumchloridlösung gewaschen. Nach dem Trocknen über Natriumsulfat wird das Lösungsmittel am Rotationsverdampfer im Vacuum bei max. 30°C abdestilliert.
Rohausbeute: 585 mg.

Nach Säulenchromatographie mit Diethylether/Diisopropylether/Hexan (1 : 1 : 1) werden 523,6 mg der Titelverbindung in Form eines farblosen Öls erhalten.

| | |
|---|---|
| $^1H$-NMR($CDCl_3$): | 0,6 - 1,0 (m,3H) |
| | 3,57 (s,3H) |
| | 3,35 - 3,87 (m,1H) |
| | 4,1 - 5,05 (m,4H) |
| | 7,2 - 7,63 (m,6H) |
| | 7,7 - 8,12 (m,4H) |

Beispiel 14

3α,ß-(1RS-1-Ethyl-pentan-2-on-1-yl)-6ß-(3RS-acetoxy-n-octyl)-7α-benzoyloxy-2-oxabicyclo[3.3.0]octan

Zur Lösung von 400 mg 3α,ß-Acetoxy-6ß-(3RS-acetoxy-n-octyl)-7α-benzoyloxy-2-oxabicyclo[3.3.0]octan und 162 mg 4-Trimethylsilyloxy-hept-3-en in 5 ml absolutem Methylenchlorid wird unter Eiskühlung und Rühren die Lösung von 0,1 ml Zinn-IV-chlorid in 1 ml absolutem Methylenchlorid tropfenweise zugefügt. Der Reaktionsverlauf wird dünnschichtchromatographisch verfolgt (Diisopropylether/Hexan 4 : 1). Nach 30 Minuten wird das Reaktionsgemisch entsprechend der in Beispiel 13 beschriebenen Weise aufgearbeitet, wobei man 391,4 mg Rohprodukt erhält, dessen Säulenchromatographie mit Diisopropylether/Hexan (4 : 1) 313,6 mg der Titelverbindung in Form eines farblosen Öls liefert.

| | |
|---|---|
| $^{1}H$-NMR($CDCl_3$): | 0,6 - 1,03 (m,9H) |
| | 2,0 (s,3H) |
| | 3,9 - 5,07 (m,4H) |
| | 7,23 - 7,6 (m,3H) |
| | 7,7 - 8,15 (m,2H) |
| $^{13}C$-NMR (Acetonitril-$d_3$): | 74,74; 74,98; 79,12; 80,4; 81,01; 82,53; 82,96; 167,07; 172,12; 214,42; 214,85. |

Die Darstellung des 4-Trimethylsilyloxy-hept-3-ens erfolgt analog R. D. Miller et al., Synthesis 1979, 730 durch Umsetzung von 4-Heptanon mit Trimethyljodsilan in Gegenwart von Hexamethyldisilazan in Pentan. Aus 11,4 g 4-Heptanon werden 14,77 g 4-Trimethylsilyloxy-hept-3-en erhalten, $Sdp._{0,3}$ 29°C.

| | | |
|---|---|---|
| $^1H$-NMR($CDCl_3$): | 0,24 | (s,9H) |
| | 0,7 - 1,1 | (m,6H) |
| | 4,24 - 4,75 | (m,1H) |

Beispiel 15

3α,ß-(1,1-Bis-ethoxycarbonyl-ethyl-1)-6ß-(3RS-acetoxy-n-octyl)-7α-benzoyloxy-2-oxabicyclo[3.3.0]octan

461 mg 3α,ß-Acetoxy-6ß-(3RS-acetoxy-n-octyl)-7α-benzoyloxy-2-oxabicyclo[3.3.0]octan und 493 mg 3-Trimethylsilyloxy-3-ethoxy-2-methylacrylsäureethylester werden entsprechend der in Beispiel 13 beschriebenen Verfahrensweise mit Hilfe von Zinn-IV-chlorid unter Eiskühlung zur Reaktion gebracht und nach 45 Minuten aufgearbeitet (Dünnschichtchromatographie mit Diethylether/Hexan 1 : 1).

Das Reaktionsprodukt wird mit Diisopropylether chromatographiert, wobei 350 mg der Titelverbindung in Form eines farblosen Öls erhalten werden.

| | | |
|---|---|---|
| $^1H$-NMR($CDCl_3$): | 0,55 - 1,0 | (m,3H) |
| | 1,44 | (s,3H) |
| | 2,0 | (s,3H) |
| | 3,87 - 4,35 | (q,4H) |
| | 4,35 - 5,1 | (m,4H) |
| | 7,2 - 7,51 | (m,3H) |
| | 7,8 - 8,1 | (m,2H) |

Die Darstellung des 3-Trimethylsilyloxy-3-ethoxy-2-methylacrylsäureethylesters erfolgt analog C. Ainsworth et al., J.Organometal.Chem. 46, 59 (1972), durch Umsetzung von Methylmalonsäurediethylester mit Natriumhydrid und Trimethylchlorsilan in absolutem Dimethoxyethan unter Stickstoff; Sdp.$_{0,25}$60°C.

$^1H$-NMR($CDCl_3$):

| | | |
|---|---|---|
| 0,31 | | (s,9H) |
| 1,03 | - 1,47 | (t,6H) |
| 1,56; | 1,75 | (2s,3H) |
| 3,78 | - 4,38 | (q,4H) |

Beispiel 16

3α,ß-(1-Methoxycarbonyl-1-methyl-ethyl-1)-6ß-(3RS-acetoxy-n-octyl)-7α-benzoyloxy-2-oxabicyclo[3.3.0]octan

Zur Lösung von 460,6 mg 3α,ß-Acetoxy-6ß-(3RS-acetoxy-n-octyl)-7α-benzoyloxy-2-oxabicyclo[3.3.0]octan in 5 ml absolutem Methylenchlorid werden unter Eiskühlung und Rühren 0,50 ml 1-Trimethylsilyloxy-1-methoxy-2,2-dimethylethen (C. Ainsworth et al., J.Organometal.Chem. 46, 59 (1972)) und eine Lösung von 0,04 ml Zinn-IV-chlorid in 2 ml absolutem Methylenchlorid gegeben. Das Dünnschichtchromatogramm (Laufmittel: Diethylether/Hexan 1 : 1) zeigt eine sofortige Umsetzung. Das Reaktionsgemisch wird entsprechend der in Beispiel 13 beschriebenen Weise aufgearbeitet, wobei 500 mg Rohprodukt erhalten werden, das nach Säulenchromatographie mit Diethylether/Hexan (1 : 1) 300,4 mg der Titelverbindung in Form eines farblosen Öls ergibt.

$^1H$-NMR($CDCl_3$):

| | | |
|---|---|---|
| 0,64 | - 1,03 | (m,3H) |
| 1,16; | 1,22 | (2s,6H) |
| 2,0; | 2,01 | (2s ,3H) |
| 3,64 | | (s,3H) |
| 4,1 | - 5,2 | (m,4H) |
| 7,2 | - 7,54 | (m,3H) |
| 7,8 | - 8,12 | (m,2H) |

Beispiel 17

3α,ß-(4-Ethoxycarbonyl-2-oxo-3,3-dimethyl-butyl-1)-6ß-(3RS-acetoxy-n-octyl)-7α-benzoyloxy-2-oxabicyclo[3.3.0]octan

a) 6,18 g 3,3-Dimethyl-laevulinsäureethylester wurden analog R. D. Miller et al., Synthesis 1979, 730 silyliert, wobei 7 g 1-Ethoxycarbonyl-2,2-dimethyl-3-trimethylsilyloxy-but-3-en vom Siedepunkt 106 - 109°C bei 20 mm anfielen. Da das Destillat noch Ausgangsmaterial enthielt, wurde es vor dem weiteren Gebrauch durch Säulenchromatographie mit Diethylether/Hexan (1 : 1) gereinigt.

| | | |
|---|---|---|
| $^1H$-NMR($CDCl_3$): | 1,24 | (s,9H) |
| | 2,25 - 2,47 | (d,2H) |
| | 3,85 - 4,27 | (q+2d,4H) |

b) Zur Lösung von 172,1 mg 3α,ß-Acetoxy-6ß-(3RS-acetoxy-n-octyl)-7α-benzoyloxy-2-oxabicyclo[3.3.0]octan und 93,1 mg 1-Ethoxycarbonyl-2,2-dimethyl-3-trimethylsilyloxy-but-3-en in 3 ml absolutem Toluol wird unter Eiskühlung und Rühren ein Tropfen Bortrifluorid-Etherat-Lösung (50 % $BF_3$ in Diethylether) gegeben. Es wird eine Stunde unter Eiskühlung und eine Stunde bei Raumtemperatur gerührt und der Reaktionsverlauf dünnschichtchromatographisch verfolgt (Laufmittel: Diethylether/Hexan - 1 : 1). Die Aufarbeitung erfolgt analog Beispiel 13.

Nach Säulenchromatographie mit Diethylether/Hexan (1 : 1) werden 46,6 mg der Titelverbindung in Form eines farblosen Öls erhalten. In weiteren Fraktionen sind noch 16,9 mg mit leichten Verunreinigungen enthalten.

$^{1}H$-NMR($CDCl_3$):

| | | | |
|---|---|---|---|
| 0,6 | - 1,01 | (m,3H) |
| 1,24 | | (s) |
| 2,0; | 2,01 | (2s,3H) |
| 3,84 | - 5,15 | (m+q,6H) |
| 7,23 | - 7,55 | (m,3H) |
| 7,8 | - 8,1 | (m,2H) |

Beispiel 18

Zur Lösung von 540,5 mg 3α,ß-Acetoxy-6ß-(3RS-acetoxy-n-octyl)-7α-benzoyloxy-2-oxabicyclo[3.3.0]octan und 286,8 mg 1-Ethoxycarbonyl-2,2-dimethyl-3-trimethylsilyloxy-but-3-en in 10 ml absolutem Toluol wird unter Eiskühlung und Rühren die Lösung von 0,138 ml Zinn-IV-chlorid in 10 ml absolutem Toluol gegeben. Man verfährt weiter wie in Beispiel 17b und erhält so 347,7 mg des (auch spektroskopisch) gleichen Produktes wie in Beispiel 17b in Form eines farblosen Öls.

Beispiel 19

3α,ß-(1RS-2-Ethoxycarbonyl-1-acetyl-ethyl-1)-6ß-(3RS-acetoxy-n-octyl)-7α-benzoyloxy-2-oxabicyclo[3.3.0]octan

Zur Lösung von 558,6 mg 3α,ß-Acetoxy-6ß-(3RS-acetoxy-n-octyl)-7α-benzoyloxy-2-oxabicyclo[3.3.0]octan und 524,8 mg 1-Ethoxycarbonyl-3-trimethylsilyloxy-but-2-en in 10 ml absolutem Toluol wird unter Eiskühlung und Rühren die Lösung von 0,14 ml Zinn-IV-chlorid in 10 ml absolutem Toluol zugetropft. Der Reaktionsverlauf wird dünnschichtchromatographisch verfolgt (Diethylether/Hexan 1 : 1). Nach Zugabe des Zinn-IV-chlorids wird noch 2 Stunden nachgerührt und dann analog Beispiel 13 aufgearbeitet.
Rohausbeute: 655,8 mg.

Nach Säulenchromatographie mit Diethylether/Hexan (10 : 1)

werden 186,5 mg der Titelverbindung in Form eines farblosen Öls erhalten sowie in einer weiteren Fraktion 63,9 mg leicht verunreinigtes Produkt.

| | | |
|---|---|---|
| $^1H$-NMR($CDCl_3$): | 0,6 - 1,0 | (m,3H) |
| | 2,0; 2,01 | (2s,3H) |
| | 2,27; 2,29 | (2s,3H) |
| | 3,42 - 3,88 | (m,1H) |
| | 3,75 - 5,13 | (m+q,6H) |
| | 7,2 - 7,55 | (m,3H) |
| | 7,75 - 8,1 | (m,2H) |

Die Darstellung des 1-Ethoxycarbonyl-3-trimethylsilyloxy-but-2-en erfolgt analog I. Fleming et al., Synthesis 1979, 736, durch Umsetzung von Laevulinsäureethylester mit Lithiumdiisopropylamid und Trimethylchlorsilan in absolutem Tetrahydrofuran in der Kälte, $Sdp_{18}$ 100 - 105°C.

| | | |
|---|---|---|
| $^1H$-NMR($CDCl_3$): | 0,22 | (s,9H) |
| | 1,05 - 1,45 | (t,3H) |
| | 1,79 | (d,3H) |
| | 2,90 - 3,12 | (d,2H) |
| | 3,87 - 4,33 | (q,2H) |
| | 4,44 - 4,77 | (t,1H) |

Patentansprüche

1) Verfahren zur Herstellung von 2-Oxa-bicyclo[3.3.0] octanderivaten der allgemeinen Formel

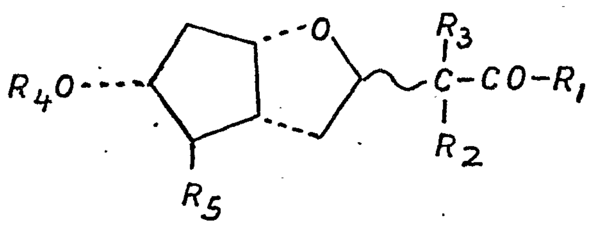

I

worin

$R_1$ einen Alkylrest mit 1 bis 5 Kohlenstoffatomen, vorzugsweise einen Methyl- oder Ethylrest, oder die Gruppe

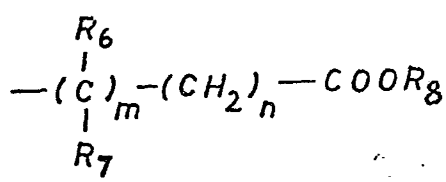

worin $R_6$ und $R_7$ gleich oder verschieden sind und für Wasserstoff oder Alkylreste mit 1 bis 4 Kohlenstoffatomen oder gemeinsam für die Gruppe $-(CH_2)_p-$ stehen und m eine der Zahlen Null oder 1, n eine der Zahlen Null, 1, 2 oder 3 und p eine der Zahlen 4, 5 oder 6 ist und $R_8$ einen leicht abspaltbaren Esterrest bedeutet,

oder einen Rest $-(CH_2)_q-OR_9$, in dem q eine der Zahlen 1, 2 oder 3 ist und $R_9$ eine Acetyl-, Benzoyl-, Trimethylsilyl-, tert.Butyl-dimethylsilyl- oder tert.Butyl-diphenylsilyl-Gruppe ist,

oder einen gegebenenfalls durch eine Methyl-, Ethyl- Trifluormethyl- oder Methoxygruppe oder durch eine Gruppe $OR_9$, in der $R_9$ die gleiche Bedeutung wie vorstehend hat, durch Fluor, Chlor oder Brom oder durch die Gruppe

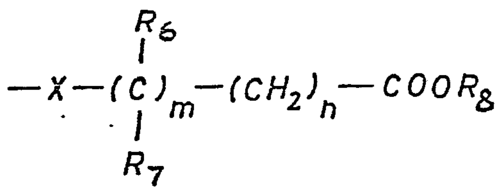

worin X eine einfache Bindung oder ein Sauerstoffatom ist und $R_6$, $R_7$, $R_8$, m und n die gleiche Bedeutung wie vorstehend haben, substituierten Phenylrest,

oder eine Methoxy-, Ethoxy- oder Trimethylsilyloxygruppe,

oder zusammen mit $R_3$ die Gruppe $-(CH_2)_r-$, in der r eine ganze Zahl von 3 bis 5 ist,

bedeutet,

$R_2$ ein Wasserstoffatom, einen Alkylrest mit 1 bis 4 Kohlenstoffatomen oder die Gruppe $-(CH_2)_s-COOR_8$, in der $R_8$ die gleiche Bedeutung wie oben hat und s für Null, 1 oder 2 steht, darstellt,

$R_3$ ein Wasserstoffatom, einen Alkylrest mit 1 bis 4 Kohlenstoffatomen oder gemeinsam mit $R_1$ die oben (bei $R_1$) definierte Gruppe bedeutet,

$R_4$ für einen Acylrest, einen Tri-nieder-alkylsilylrest, einen tert.Butyl-diphenylsilylrest oder für einen Triphenylsilylrest steht, und

$R_5$ die Gruppe $-CH_2-O-R_4'$ oder die Gruppe

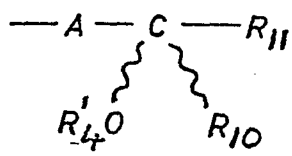

worin $R_4'$ jeweils im Rahmen der Definition von $R_4$ gleich oder verschieden zu $R_4$ ist und worin A für $-CH_2-CH_2-$, (trans)-CH=CH- oder für -C≡C- steht, $R_{10}$ für Wasserstoff, Methyl-, Ethyl- oder Trifluormethyl steht und $R_{11}$ einen Alkylrest

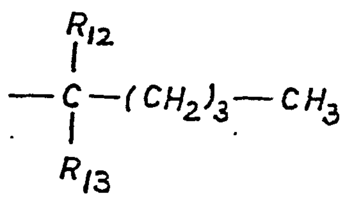

in dem $R_{12}$ und $R_{13}$ gleich oder verschieden sind und für Wasserstoff, Methyl oder Ethyl stehen, oder einen Cyclohexylrest

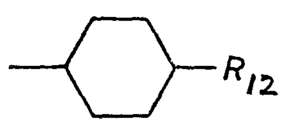

in dem $R_{12}$ die gleiche Bedeutung wie vorstehend hat, bedeutet, darstellt,

dadurch gekennzeichnet, daß man in einem inerten Lösungsmittel in Gegenwart von einer oder mehren Lewis-Säuren eine Verbindung der Formel

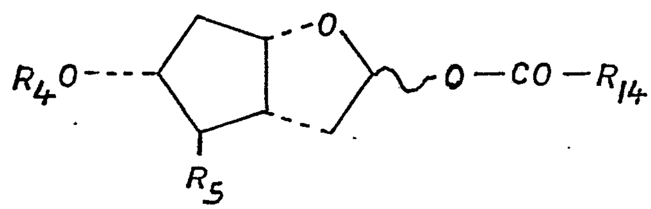

II

worin $R_4$ und $R_5$ die gleiche Bedeutung wie in Formel I haben und $R_{14}$ für einen Alkylrest mit 1 bis 4 Kohlenstoffatomen oder für einen Phenylrest steht, mit einer Verbindung der Formel

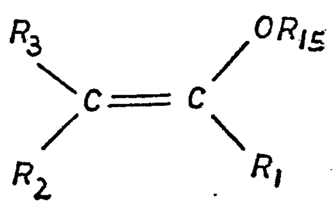

III

worin $R_1$, $R_2$ und $R_3$ die gleiche Bedeutung wie in Formel I haben und $R_{15}$ für eine Trimethylsilyl- oder eine niedere Alkanoylgruppe steht, bei Temperaturen zwischen -80°C und +60°C umsetzt.

2) Verfahren zur Herstellung von Verbindungen der Formel

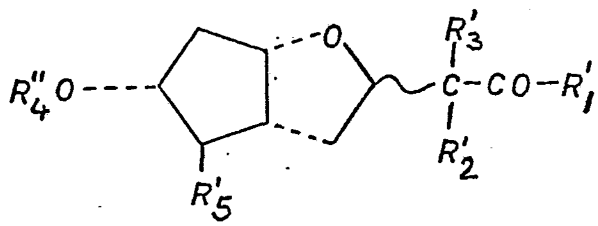

Ia

worin

$R_1'$ einen Methyl- oder Ethylrest
oder die Gruppe

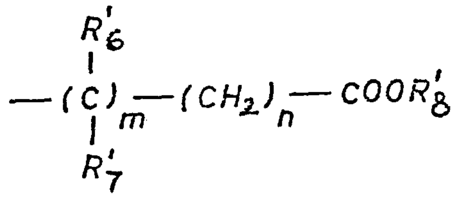

in der $R_6'$ und $R_7'$ gleich oder verschieden sind und für Wasserstoffatome oder Methylgruppen stehen, m eine der Zahlen Null oder 1, n eine der Zahlen Null, 1, 2 oder 3 und $R_8'$ einen Methyl- oder Ethylrest bedeutet,

oder einen gegebenenfalls durch eine Methyl-, Ethyl-, Trifluormethyl- oder Methoxygruppe oder durch eine Gruppe $OR_9$, in der $R_9$ eine Acetyl-, Benzoyl-, Trimethylsilyl-, tert.Butyl-dimethylsilyl- oder tert. Butyl-diphenyl-silyl-Gruppe ist, durch ein Fluor-, Chlor- oder Bromatom oder durch die Gruppe

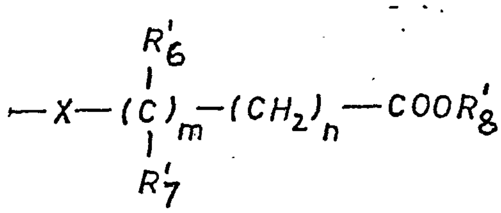

worin X eine einfache Bindung oder ein Sauerstoffatom ist und $R_6'$, $R_7'$, $R_8'$, m und n die gleiche Bedeutung wie vorstehend haben, substituierten Phenylrest

oder eine Methoxy-, Ethoxy- oder Trimethylsilyloxygruppe

oder zusammen mit $R_3'$ die Gruppe $-(CH_2)_r-$, in der r eine ganze Zahl von 3 bis 5 ist, bedeutet,

$R_2'$ ein Wasserstoffatom, einen Methylrest oder die Gruppe $-(CH_2)_s-COOR_8$, in der $R_8$ die gleiche Bedeutung wie oben hat und s für Null, 1 oder 2 steht, darstellt,

$R_3'$ ein Wasserstoffatom, einen Methylrest oder gemeinsam mit $R_1'$ die oben (bei $R_1'$) definierte Gruppe $-(CH_2)_r-$ bedeutet,

$R_4''$ für einen niederen Alkanoylrest mit 1 bis 5 Kohlenstoffatomen, insbesondere den Acetylrest, oder einen Benzoyl- oder 4-Phenyl-benzoylrest, einen Trimethylsilyl-, tert.Butyl-dimethylsilyl- oder einen tert.Butyl-diphenylsilylrest steht und

$R_5'$ die Gruppe $-CH_2-O-R_4'''$ oder die Gruppe

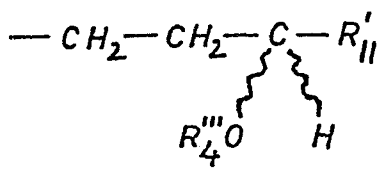

worin $R_4'''$ jeweils im Rahmen der Definition von $R_4''$ gleich oder verschieden zu $R_4''$ ist und $R_{11}'$ für einen n-Pentyl- oder für einen Cyclohexylrest steht, bedeutet,

gemäß Anspruch 1, dadurch gekennzeichnet, daß man in einem inerten Lösungsmittel in Gegenwart von einer oder mehreren Lewis-Säuren eine Verbindung der Formel

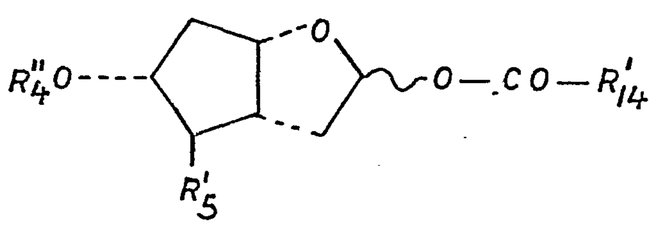

IIa

worin $R_4''$ und $R_5'$ die gleiche Bedeutung wie oben haben und $R_{14}'$ für einen Alkylrest mit 1 bis 4 Kohlenstoffatomen, insbesondere einen Methylrest, oder für einen Phenylrest steht,

mit einer Verbindung der Formel

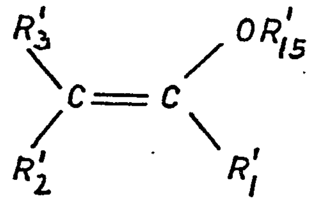

III a

worin $R_1'$, $R_2'$ und $R_3'$ die gleiche Bedeutung wie oben haben und $R_{15}'$ für eine Trimethylsilyl- oder eine Acetylgruppe, steht, bei Temperaturen zwischen -80°C und +60°C umsetzt.

3) Verfahren zur Herstellung von Verbindungen der Formel

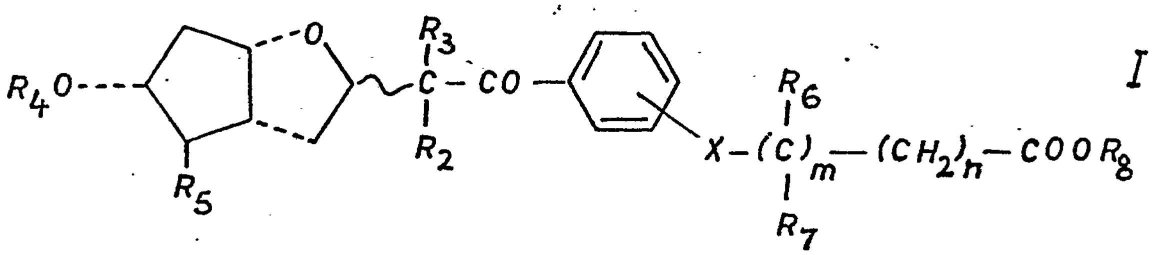

Ib

in der $R_2$ bis $R_8$, X sowie m und n die gleiche Bedeutung wie in Anspruch 1 haben, gemäß Ansprüchen 1 und 2, dadurch gekennzeichnet, daß man die Verbindung der Formel II in einem inerten Lösungsmittel in Gegenwart von einer oder mehreren Lewis-Säuren mit einer Verbindung der Formel

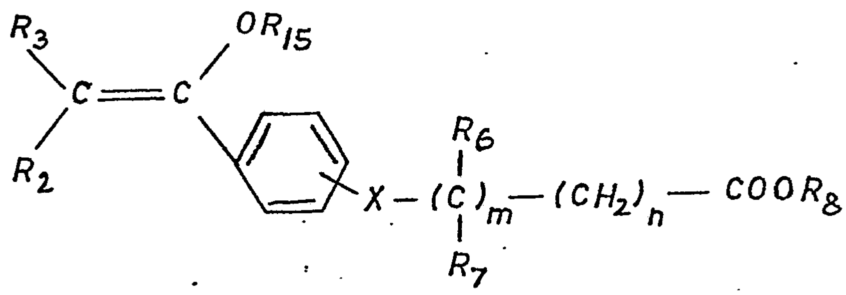

III b

worin $R_2$, $R_3$, $R_6$ bis $R_8$, $R_{15}$, X sowie m und n die gleiche Bedeutung wie in Anspruch 1 haben, bei Temperaturen zwischen -80°C und +60°C umsetzt.

4) Verfahren zur Herstellung von Verbindungen der Formel

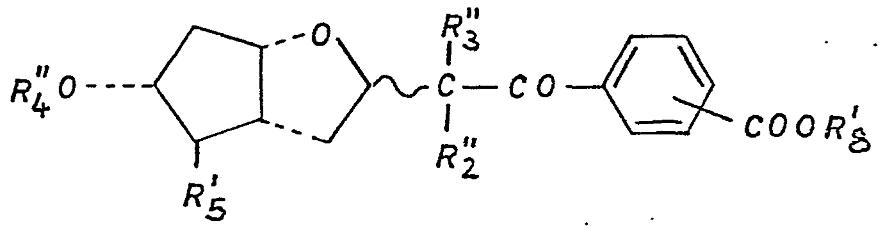

worin $R_4''$, $R_5'$ und $R_8'$ die gleiche Bedeutung wie in Anspruch 2 haben und $R_2''$ und $R_3''$ gleich oder verschieden sind und für Wasserstoffatome oder Methylgruppen stehen, gemäß Ansprüchen 1 bis 3, dadurch gekennzeichnet, daß man die Verbindung der Formel IIa in einem inerten Lösungsmittel in Gegenwart von einer oder mehreren Lewis-Säuren mit einer Verbindung der Formel

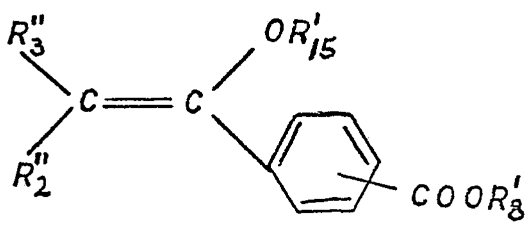

III c

worin $R_2''$, $R_3''$, $R_8'$ und $R_{15}'$ die gleiche Bedeutung wie oben bzw. wie in Anspruch 2 haben, bei Temperaturen zwischen -80°C und +60°C umsetzt.

5) Verfahren gemäß Ansprüchen 1 bis 4, dadurch gekennzeichnet, daß Lewis-Säuren aus der Gruppe Aluminiumchlorid, Bortrifluorid, Kupfer-II-sulfat, Silberperchlorat, Silbertetrafluoroborat, Titan-IV-chlorid, Zinkbromid, Zinkchlorid oder Zinn-IV-chlorid Verwendung finden.

6) Verfahren gemäß Ansprüchen 1 bis 5, dadurch gekennzeichnet, daß als Lewis-Säuren Bortrifluorid oder Zinn-IV-chlorid eingesetzt werden.

7) Verfahren gemäß Ansprüchen 1 - 6, dadurch gekennzeichnet, daß die Lewis-Säuren in Mengen von $10^{-3}$ Mol bis $10^{2}$ Mol, vorzugsweise in äquimolaren Mengen bezogen auf die molaren Mengen an eingesetzter Verbindung der allgemeinen Formel II bzw. IIa zur Anwendung kommen.

8) Verfahren gemäß Ansprüchen 1 bis 7, dadurch gekennzeichnet, daß die Reaktion bei Temperaturen zwischen -5°C und +20°C in einem niederen chlorierten aliphatischen Kohlenwasserstoff, insbesondere Methylenchlorid, durchgeführt wird.

9) 2-Oxa-bicyclo[3.3.0]octanderivate der allgemeinen Formel

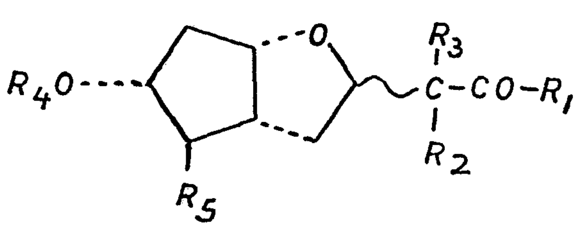

I

worin

$R_1$ einen Alkylrest mit 1 bis 5 Kohlenstoffatomen, vorzugsweise einen Methyl- oder Ethylrest, oder die Gruppe

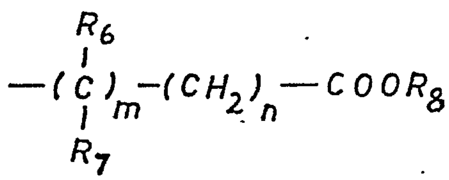

worin $R_6$ und $R_7$ gleich oder verschieden sind und für Wasserstoff oder Alkylreste mit 1 bis 4 Kohlenstoffatomen oder gemeinsam für die Gruppe $-(CH_2)_p-$ stehen und m eine der Zahlen Null oder 1, n eine der Zahlen Null, 1, 2 oder 3 und p eine der Zahlen 4, 5 oder 6 ist und $R_8$ einen leicht abspaltbaren Esterrest bedeutet,

oder einen Rest $-(CH_2)_q-OR_9$, in dem q eine der Zahlen 1, 2 oder 3 ist und $R_9$ eine Acetyl-, Benzoyl-, Trimethylsilyl-, tert.Butyl-dimethylsilyl- oder tert.Butyl-diphenylsilyl-Gruppe ist,

oder einen gegebenenfalls durch eine Methyl-, Ethyl-, Trifluormethyl- oder Methoxygruppe oder durch eine Gruppe $OR_9$, in der $R_9$ die gleiche Bedeutung wie vorstehend hat, durch Fluor, Chlor oder Brom oder durch die Gruppe

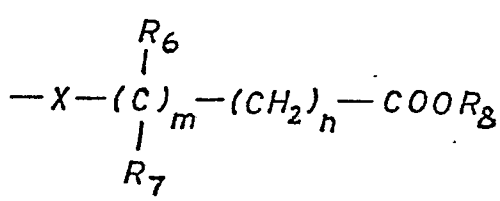

worin X eine einfache Bindung oder ein Sauerstoffatom ist und $R_6$, $R_7$, $R_8$, m und n die gleiche Bedeutung wie vorstehend haben, substituierten Phenylrest

oder eine Methoxy-, Ethoxy- oder Trimethylsilyloxygruppe

oder zusammen mit $R_3$ die Gruppe $-(CH_2)_r-$, in der r eine ganze Zahl von 3 bis 5 ist,

bedeutet,

$R_2$ ein Wasserstoffatom, einen Alkylrest mit 1 bis 4 Kohlenstoffatomen oder die Gruppe $-(CH_2)_s-COOR_8$, in der $R_8$ die gleiche Bedeutung wie oben hat und s für Null, 1 oder 2 steht, darstellt,

$R_3$ ein Wasserstoffatom, einen Alkylrest mit 1 bis 4 Kohlenstoffatom oder gemeinsam mit $R_1$ die oben (bei $R_1$) definierte Gruppe $-(CH_2)_r-$ bedeutet,

$R_4$ für einen Acylrest oder einen Tri-nieder-alkylsilyl-, einen tert.Butyl-diphenylsilyl- oder für einen Triphenylsilylrest steht, und

$R_5$ die Gruppe $-CH_2-O-R_4'$ oder die Gruppe

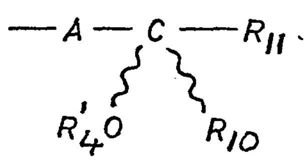

worin $R_4'$ jeweils im Rahmen der Definition von $R_4$ gleich oder verschieden zu $R_4$ ist und worin A für $-CH_2-CH_2-$, (trans)-CH=CH- oder für -C≡C- steht, $R_{10}$ für Wasserstoff, Methyl-, Ethyl- oder Trifluormethyl steht und $R_{11}$ einen Alkylrest

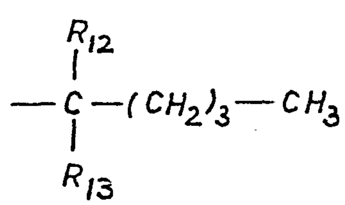

in dem $R_{12}$ und $R_{13}$ gleich oder verschieden sind und für Wasserstoff, Methyl oder Ethyl stehen, oder einen Cyclohexylrest

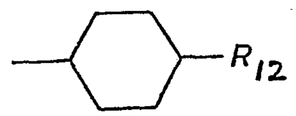

in dem $R_{12}$ die gleiche Bedeutung wie vorstehend hat,
bedeutet,
in Form der reinen Isomeren oder deren Gemischen, sofern sie nach dem Verfahren der Ansprüche 1 - 8 oder dessen Äquivalenten erhalten wurden.

10) Neue 2-Oxa-bicyclo[3.3.0]octanderivate der allgemeinen Formel

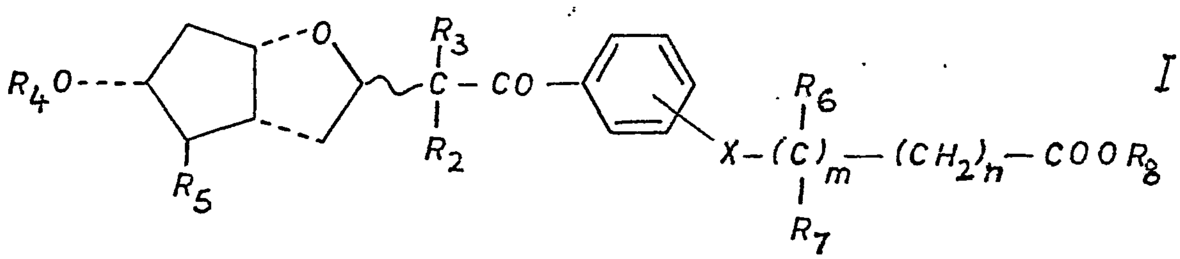

Ib

in der $R_2$ bis $R_8$, X sowie m und n die gleiche Bedeutung wie in Anspruch 9 haben,
in Form der reinen Isomeren oder deren Gemischen.

11) Neue 2-Oxa-bicyclo[3.3.0]octanderivate der allgemeinen Formel

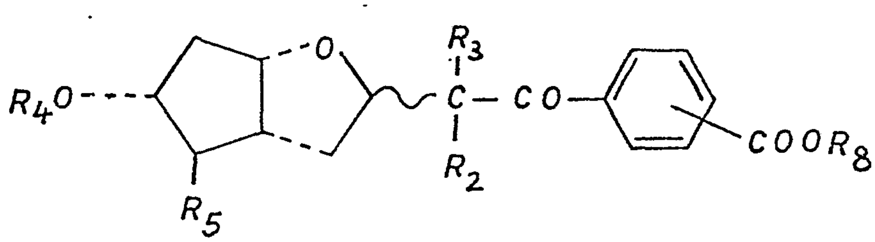

worin $R_2$ bis $R_5$ und $R_8$ die gleiche Bedeutung wie in Anspruch 9 haben,
in Form der reinen Isomeren oder deren Gemischen.

12) Neue 2-Oxa-bicyclo[3.3.0]octanderivate der allgemeinen Formel

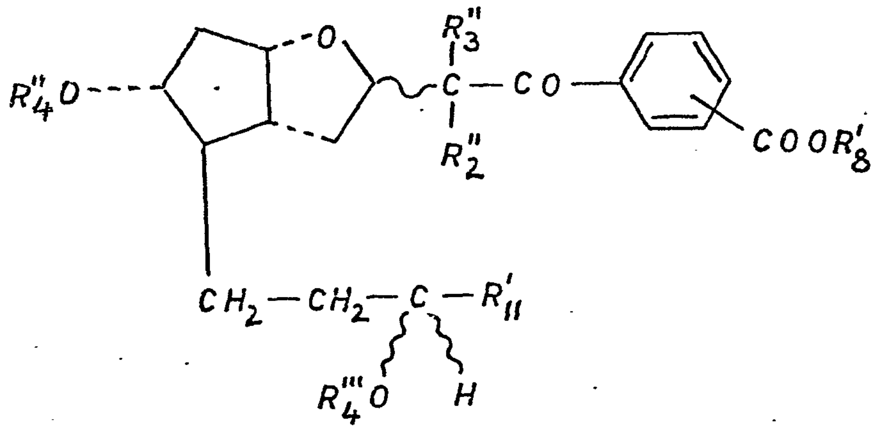

Ic

worin $R_2''$ und $R_3''$ gleich oder verschieden sind und Wasserstoffatome oder Methylgruppen bedeuten, $R_4''$ und $R_4'''$ gleich oder verschieden sind und jeweils für einen niederen Alkanoylrest mit 1 bis Kohlenstoffatomen, insbesondere Acetyl, oder einen Benzoyl- oder 4-Phenylbenzoylrest oder für einen Trimethylsilyl-, tert. Butyl-dimethylsilyl- oder tert.Butyl-diphenylsilylrest stehen, $R_8'$ einen Methyl- oder Ethylrest darstellt und $R_{11}'$ für einen n-Pentyl- oder für einen Cyclohexylrest steht

in Form der reinen Isomeren oder deren Gemischen.

Patentansprüche für Österreich

1) Verfahren zur Herstellung von 2-Oxa-bicyclo[3.3.0] octanderivaten der allgemeinen Formel

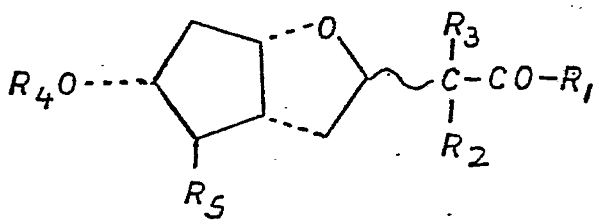

I

worin

$R_1$ einen Alkylrest mit 1 bis 5 Kohlenstoffatomen, vorzugsweise einen Methyl- oder Ethylrest, oder die Gruppe

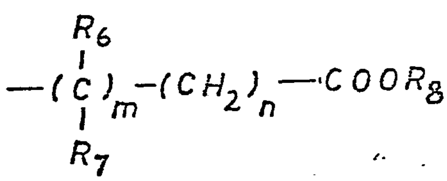

worin $R_6$ und $R_7$ gleich oder verschieden sind und für Wasserstoff oder Alkylreste mit 1 bis 4 Kohlenstoffatomen oder gemeinsam für die Gruppe $-(CH_2)_p-$ stehen und m eine der Zahlen Null oder 1, n eine der Zahlen Null, 1, 2 oder 3 und p eine der Zahlen 4, 5 oder 6 ist und $R_8$ einen leicht abspaltbaren Esterrest bedeutet,

oder einen Rest $-(CH_2)_q-OR_9$, in dem q eine der Zahlen 1, 2 oder 3 ist und $R_9$ eine Acetyl-, Benzoyl-, Trimethylsilyl-, tert.Butyl-dimethylsilyl- oder tert.Butyl-diphenylsilyl-Gruppe ist,

oder einen gegebenenfalls durch eine Methyl-, Ethyl- Trifluormethyl- oder Methoxygruppe oder durch eine Gruppe $OR_9$, in der $R_9$ die gleiche Bedeutung wie vorstehend hat, durch Fluor, Chlor oder Brom oder durch die Gruppe

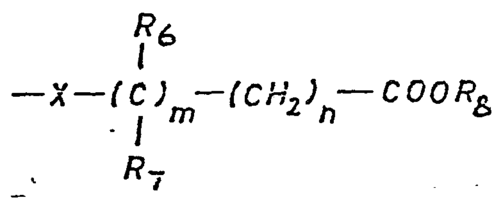

worin X eine einfache Bindung oder ein Sauerstoffatom ist und $R_6$, $R_7$, $R_8$, m und n die gleiche Bedeutung wie vorstehend haben, substituierten Phenylrest,

oder eine Methoxy-, Ethoxy- oder Trimethylsilyloxygruppe,

oder zusammen mit $R_3$ die Gruppe $-(CH_2)_r-$, in der r eine ganze Zahl von 3 bis 5 ist,

bedeutet,

$R_2$ ein Wasserstoffatom, einen Alkylrest mit 1 bis 4 Kohlenstoffatomen oder die Gruppe $-(CH_2)_s-COOR_8$, in der $R_8$ die gleiche Bedeutung wie oben hat und s für Null, 1 oder 2 steht, darstellt,

$R_3$ ein Wasserstoffatom, einen Alkylrest mit 1 bis 4 Kohlenstoffatomen oder gemeinsam mit $R_1$ die oben (bei $R_1$) definierte Gruppe bedeutet,

$R_4$ für einen Acylrest, einen Tri-nieder-alkylsilylrest, einen tert.Butyl-diphenylsilylrest oder für einen Triphenylsilylrest steht, und

$R_5$ die Gruppe $-CH_2-O-R_4'$ oder die Gruppe

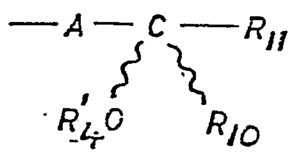

worin $R_4'$ jeweils im Rahmen der Definition von $R_4$ gleich oder verschieden zu $R_4$ ist und worin A für $-CH_2-CH_2-$, (trans)-CH=CH- oder für -C≡C- steht, $R_{10}$ für Wasserstoff, Methyl-, Ethyl- oder Trifluormethyl steht und $R_{11}$ einen Alkylrest

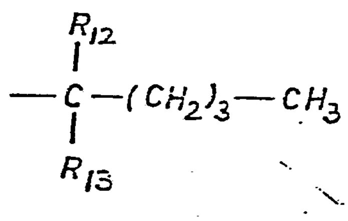

in dem $R_{12}$ und $R_{13}$ gleich oder verschieden sind und für Wasserstoff, Methyl oder Ethyl stehen, oder einen Cyclohexylrest

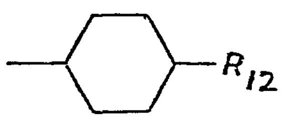

in dem $R_{12}$ die gleiche Bedeutung wie vorstehend hat, bedeutet, darstellt,

dadurch gekennzeichnet, daß man in einem inerten Lösungsmittel in Gegenwart von einer oder mehren Lewis-Säuren eine Verbindung der Formel

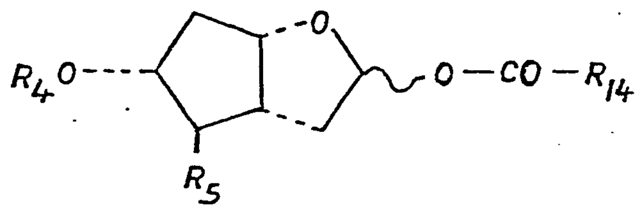

II

worin $R_4$ und $R_5$ die gleiche Bedeutung wie in Formel I haben und $R_{14}$ für einen Alkylrest mit 1 bis 4 Kohlenstoffatomen oder für einen Phenylrest steht, mit einer Verbindung der Formel

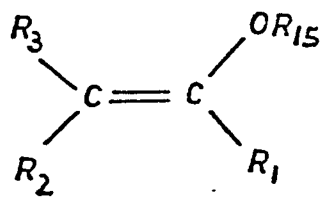

III

worin $R_1$, $R_2$ und $R_3$ die gleiche Bedeutung wie in Formel I haben und $R_{15}$ für eine Trimethylsilyl- oder eine niedere Alkanoylgruppe steht, bei Temperaturen zwischen -80°C und +60°C umsetzt.

2) Verfahren zur Herstellung von Verbindungen der Formel

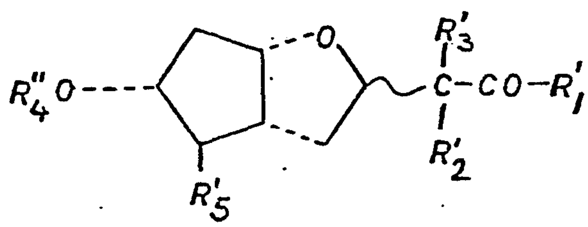

Ia

worin

$R_1'$ einen Methyl- oder Ethylrest
oder die Gruppe

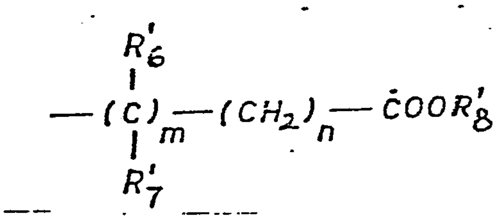

in der $R'_6$ und $R'_7$ gleich oder verschieden sind und für Wasserstoffatome oder Methylgruppen stehen, m eine der Zahlen Null oder 1, n eine der Zahlen Null, 1, 2 oder 3 und $R'_8$ einen Methyl- oder Ethylrest bedeutet,

oder einen gegebenenfalls durch eine Methyl-, Ethyl-, Trifluormethyl- oder Methoxygruppe oder durch eine Gruppe $OR_9$, in der $R_9$ eine Acetyl-, Benzoyl-, Trimethylsilyl-, tert.Butyl-dimethylsilyl- oder tert. Butyl-diphenyl-silyl-Gruppe ist, durch ein Fluor-, Chlor- oder Bromatom oder durch die Gruppe

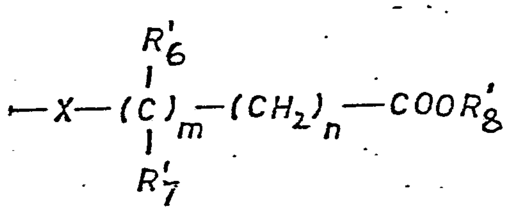

worin X eine einfache Bindung oder ein Sauerstoffatom ist und $R'_6$, $R'_7$, $R'_8$, m und n die gleiche Bedeutung wie vorstehend haben, substituierten Phenylrest

oder eine Methoxy-, Ethoxy- oder Trimethylsilyloxygruppe

oder zusammen mit $R'_3$ die Gruppe $-(CH_2)_r-$, in der r eine ganze Zahl von 3 bis 5 ist, bedeutet,

$R'_2$ ein Wasserstoffatom, einen Methylrest oder die Gruppe $-(CH_2)_s-COOR_8$, in der $R_8$ die gleiche Bedeutung wie oben hat und s für Null, 1 oder 2 steht, darstellt,

$R_3'$ ein Wasserstoffatom, einen Methylrest oder gemeinsam mit $R_1'$ die oben (bei $R_1'$) definierte Gruppe $-(CH_2)_r-$ bedeutet,

$R_4''$ für einen niederen Alkanoylrest mit 1 bis 5 Kohlenstoffatomen, insbesondere den Acetylrest, oder einen Benzoyl- oder 4-Phenyl-benzoylrest, einen Trimethylsilyl-, tert.Butyl-dimethylsilyl- oder einen tert.Butyl-diphenylsilylrest steht und

$R_5'$ die Gruppe $-CH_2-O-R_4'''$ oder die Gruppe

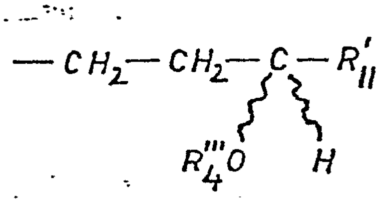

worin $R_4'''$ jeweils im Rahmen der Definition von $R_4''$ gleich oder verschieden zu $R_4''$ ist und $R_{11}'$ für einen n-Pentyl- oder für einen Cyclohexylrest steht, bedeutet,

gemäß Anspruch 1, dadurch gekennzeichnet, daß man in einem inerten Lösungsmittel in Gegenwart von einer oder mehreren Lewis-Säuren eine Verbindung der Formel

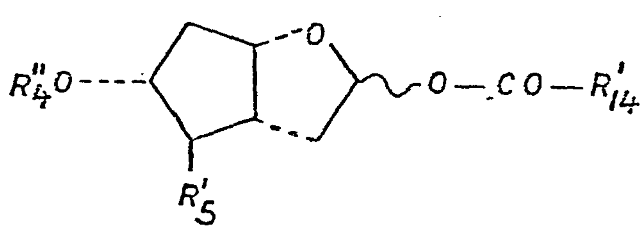

IIa

worin $R_4''$ und $R_5'$ die gleiche Bedeutung wie oben haben und $R_{14}'$ für einen Alkylrest mit 1 bis 4 Kohlenstoffatomen, insbesondere einen Methylrest, oder für einen Phenylrest steht,

mit einer Verbindung der Formel

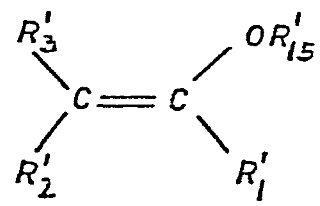

III a

worin $R'_1$, $R'_2$ und $R'_3$ die gleiche Bedeutung wie oben haben und $R'_{15}$ für eine Trimethylsilyl- oder eine Acetylgruppe, steht, bei Temperaturen zwischen -80°C und +60°C umsetzt.

3) Verfahren zur Herstellung von Verbindungen der Formel

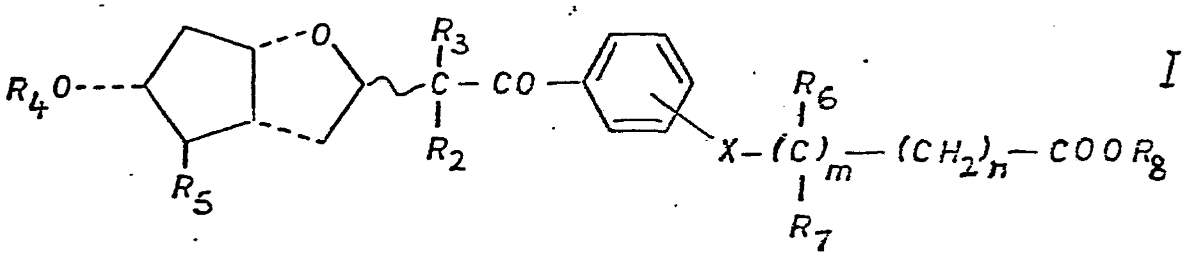

Ib

in der $R_2$ bis $R_8$, X sowie m und n die gleiche Bedeutung wie in Anspruch 1 haben, gemäß Ansprüchen 1 und 2, dadurch gekennzeichnet, daß man die Verbindung der Formel II in einem inerten Lösungsmittel in Gegenwart von einer oder mehreren Lewis-Säuren mit einer Verbindung der Formel

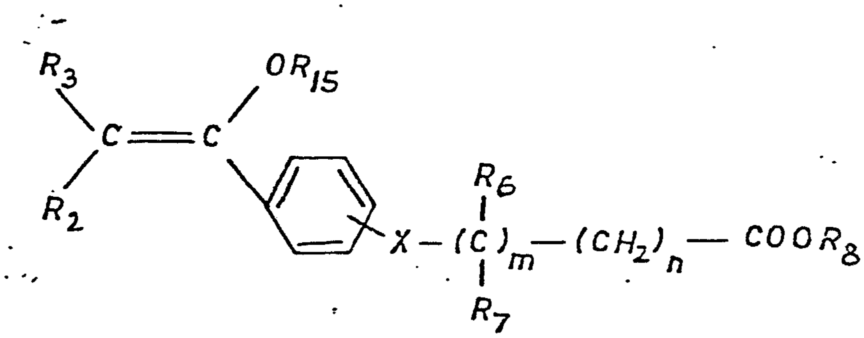

III b

worin $R_2$, $R_3$, $R_6$ bis $R_8$, $R_{15}$, X sowie m und n die gleiche Bedeutung wie in Anspruch 1 haben, bei Temperaturen zwischen -80°C und +60°C umsetzt.

4) Verfahren zur Herstellung von Verbindungen der Formel

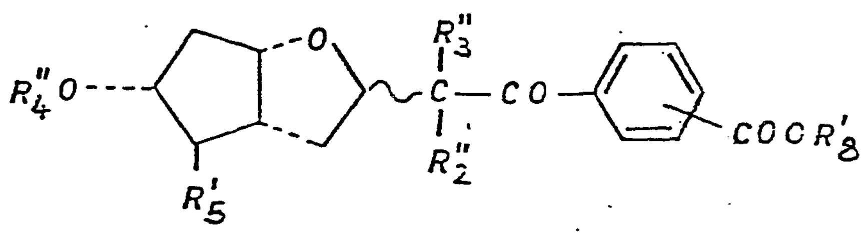

worin $R_4''$, $R_5'$ und $R_8'$ die gleiche Bedeutung wie in Anspruch 2 haben und $R_2''$ und $R_3''$ gleich oder verschieden sind und für Wasserstoffatome oder Methylgruppen stehen, gemäß Ansprüchen 1 bis 3, dadurch gekennzeichnet, daß man die Verbindung der Formel IIa in einem inerten Lösungsmittel in Gegenwart von einer oder mehreren Lewis-Säuren mit einer Verbindung der Formel

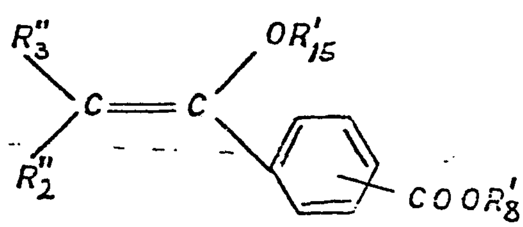

III c

worin $R_2''$ , $R_3''$ , $R_8'$ und $R_{15}'$ die gleiche Bedeutung wie oben bzw. wie in Anspruch 2 haben, bei Temperaturen zwischen -80°C und +60°C umsetzt.

5) Verfahren gemäß Ansprüchen 1 bis 4, dadurch gekennzeichnet, daß Lewis-Säuren aus der Gruppe Aluminiumchlorid, Bortrifluorid, Kupfer-II-sulfat, Silberperchlorat, Silbertetrafluoroborat, Titan-IV-chlorid, Zinkbromid, Zinkchlorid oder Zinn-IV-chlorid Verwendung finden.

6) Verfahren gemäß Ansprüchen 1 bis 5, dadurch gekennzeichnet, daß als Lewis-Säuren Bortrifluorid oder Zinn-IV-chlorid eingesetzt werden.

7) Verfahren gemäß Ansprüchen 1 - 6, dadurch gekennzeichnet, daß die Lewis-Säuren in Mengen von $10^{-3}$ Mol bis $10^{2}$ Mol, vorzugsweise in äquimolaren Mengen bezogen auf die molaren Mengen an eingesetzter Verbindung der allgemeinen Formel II bzw. IIa zur Anwendung kommen.

8) Verfahren gemäß Ansprüchen 1 bis 7, dadurch gekennzeichnet, daß die Reaktion bei Temperaturen zwischen -5°C und +20°C in einem niederen chlorierten aliphatischen Kohlenwasserstoff, insbesondere Methylenchlorid, durchgeführt wird.

0059307

Europäisches Patentamt

# EUROPÄISCHER RECHERCHENBERICHT

Nummer der Anmeldung

EP 82 10 0317

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
|---|---|---|---|
| D,A | DE-A-2 853 849 (C. ERBA)<br>----- | 1 | C 07 D 307/935 |
| | | | RECHERCHIERTE SACHGEBIETE (Int. Cl. ³) |
| | | | C 07 D 307/00 |

Der vorliegende Recherchenbericht wurde für alle Patentansprüche erstellt.

| Recherchenort | Abschlußdatum der Recherche | Prüfer |
|---|---|---|
| DEN HAAG | 16-06-1982 | BERTE M.J. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82